(19) **Europäisches Patentamt / European Patent Office / Office européen des brevets**

(11) **EP 4 578 859 A1**

(12) **EUROPEAN PATENT APPLICATION**
published in accordance with Art. 153(4) EPC

(43) Date of publication:
**02.07.2025 Bulletin 2025/27**

(21) Application number: **22957668.1**

(22) Date of filing: **06.09.2022**

(51) International Patent Classification (IPC):
**C07D 487/22** (2006.01)  **C07D 491/22** (2006.01)
**A01N 43/90** (2006.01)  **A01P 21/00** (2006.01)

(52) Cooperative Patent Classification (CPC):
**A01N 43/90; A01P 21/00; C07D 487/22;**
**C07D 491/22**

(86) International application number:
**PCT/CN2022/117298**

(87) International publication number:
**WO 2024/050694 (14.03.2024 Gazette 2024/11)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Nanjing Bostec Biological Engineering Co., Ltd**
**Nanjing, Jiangsu 210019 (CN)**

(72) Inventors:
• **REN, Yong**
  **Nanjing, Jiangsu 210019 (CN)**

• **WEI, Lihui**
  **Nanjing, Jiangsu 210019 (CN)**
• **WANG, Letian**
  **Nanjing, Jiangsu 210019 (CN)**
• **ZHOU, Dongmei**
  **Nanjing, Jiangsu 210019 (CN)**
• **MENG, Dongfeng**
  **Nanjing, Jiangsu 210019 (CN)**

(74) Representative: **Miller, Ronald Anthony**
  **76 Bramley Avenue**
  **Melbourn**
  **Royston, Hertfordshire SG8 6HG (GB)**

(54) **NATURAL PORPHIN SALT AND USE THEREOF AS PLANT GROWTH REGULATOR AND IMMUNE RESISTANCE INDUCER**

(57)    The present disclosure provides a natural porphin salt and a use thereof as a plant growth regulator and a plant immunity inducer. The porphin salt includes a salt of porphin or a salt of chlorin. Compared with the prior art, the porphin salt of the present disclosure has high stability, exhibits a stable and reliable quality during long-term storage, has a long shelf life, and is easy to carry and transport. The porphin salt has prominent water solubility, is easy to prepare into an aqueous solution with a stable content, and exhibits excellent reproducibility when applied to the field. The porphin salt can be conveniently applied at a low concentration with a strong activity and a prominent control effect. Raw materials for preparing the porphin salt are natural and widely available. The preparation of the porphin salt is emission-free, pollution-free, energy-saving, and eco-friendly. In particular, when used as a plant immunity inducer, the porphin salt exhibits a significantly better control effect than the existing products.

FIG. 1

## Description

## TECHNICAL FIELD

The present disclosure relates to a natural porphin salt and a use thereof as a plant growth regulator and a plant immunity inducer, and belongs to the technical field of plant growth regulators.

## BACKGROUND

Chlorophyll and heme are natural porphin structures produced and existing in animals and plants. Chlorophyll and heme are a material basis for life-sustaining activities such as photosynthesis and aerobic respiration in animals and plants. Heme has a classical porphin structure. Chlorophyll has a chlorin parent nucleus structure. Chlorin has more significant asymmetry and exists in more complex and diverse forms and states than porphin. Various porphin products produced through natural extraction and processing have begun to be widely used in many fields such as chemicals, medicine, food, and agriculture. For example, iron chlorin (iron chlorophyllin chloride, CN102285992B) and chlorhematin (heme, CN10048884C) have been used as novel plant growth regulators in a variety of crops. Sodium iron chlorophyllin and potassium/sodium copper chlorophyllin are used in food additives (food colorants). Protoporphyrin disodium is used for treating liver cirrhosis. In addition to commercially-available products, the Chinese patents CN101045730, CN102351867, CN102775416, CN102796108, etc. have disclosed the preparation and properties of bivalent salts such as zinc iron chlorophyllin, calcium iron chlorophyllin, magnesium iron chlorophyllin, and manganese iron chlorophyllin in recent years. In the literature (Study on Reaction Rates, Equilibrium Constants, and Structures of Metallic Salts of Iron Chlorophyllin, Master's Thesis, North China University of Science and Technology, 2017.03), the preparation of a series of metallic salts of iron chlorophyllin is investigated, including salt-producing reactions, product structures, and physical and chemical properties. Study results have shown that iron chlorophyllin can be combined with divalent ions $Mg^{2+}$, $Ca^{2+}$, $Mn^{2+}$, and $Zn^{2+}$ in a molar ratio of 1:1 to produce slightly-soluble/insoluble salts ($K_{sp} \approx 10^{-16}$ to $10^{-18}\,M^2$), and salt-producing reactions are fast (the activation energy is about 20 kJ/mol). Taking advantage of the difference in water solubility, the divalent metallic salts of these porphin chelates can be prepared through the precipitation from aqueous solutions, and the water-soluble porphin sodium is the basic raw material for preparing these products. The reported water-soluble porphin salts are mainly sodium/potassium salts of divalent transition metal-porphin chelates, such as sodium zinc chlorophyllin, potassium zinc chlorophyllin, sodium manganese chlorophyllin, and sodium copper chlorophyllin. These sodium/potassium salts have excellent solubility, and the conventional aqueous solutions of these sodium/potassium salts are strongly alkaline. However, there are few reports on sodium/potassium salts of trivalent transition metal-porphin chelates. Chlorhematin is a trivalent iron-porphin chelate, where chlorine axially binding to central iron is easily substituted with hydroxyl having a stronger coordination capacity than chlorine. Thus, sodium hydroxyhematin can be produced when an alkali and chlorhematin are added to an aqueous solution (Properties and detection methods of chlorhematin, Chinese Journal of Biochemical Pharmaceutics, 1993.66 (4): 58-59). Experiments have proved that, through the precipitation of an alkaline aqueous solution, only sodium hydroxyhematin can be prepared, but sodium chlorhematin cannot be produced. The use of sodium iron chlorophyllin and sodium copper chlorophyllin shows that these products have prominent water solubility and significantly-improved stability, and are easily transported and carried. However, there has been no report on the research of using natural porphin salts as plant growth regulators.

Plant immunity inducers, also known as plant vaccines, are a class of novel biological pesticides developed based on the vaccine engineering technology in recent years. Plant immunity inducers can regulate the metabolism and growth of plants and activate the immune systems of plants to achieve the effective prevention and control of diseases for crops (disease prevention), improve the resistance of crops, and improve the yield and quality, which is harmless to humans and animals and does not pollute the environment. Therefore, plant immunity inducers have become a hot spot for the current research and production of biological pesticides. The plant immunity inducers that have been discovered are mainly plant or microorganism-derived small molecules such as salicylic acid and matrine, large molecules such as humic acid and lentinan, and some chemically-synthesized bactericides. The research on using natural porphin salts as plant immunity inducers (plant vaccines) has not been reported.

Natural porphin has a planar parent ring and a high electron density, and is easy to chelate a metal ion to produce a chelate such as chlorophyll and heme. Alkyl or alkenyl and groups such as carboxyalkyl are linked at an outer side of a porphin ring, resulting in complicated structures and many isomers, such as acidic iron chlorin. Natural porphin is basically insoluble in water and has poor photothermal stability. Studies have shown that planar iron chlorin molecules have strong association/aggregation characteristics, and the high lipophilicity and lattice energy of the molecule as a whole makes a solid difficult to be diffused/dispersed and dissolved, i.e., $K_{sp}$ is small. In addition, the association/aggregation will be further aggravated with the extension of standing time, resulting in the further decrease in solubility, i.e., $K_{sp}$ gradually decreases. In a solution, the stability of a natural porphin compound also decreases significantly (Preliminary Study on Basic Properties of Iron Chlorin Solution, Journal of Nanjing Normal University (Natural Science Edition), 43:1, 2020, 3:

143-148). It can be seen that most of acidic porphin molecules have small polarity, strong lipid solubility, and poor water solubility, are generally insoluble or hardly-soluble in water, and exhibit poor photothermal stability under normal conditions. Although a porphin product in a solid state can have an enough shelf life when stored at a low temperature, the porphin product is easily decomposed when exposed to light in a solution, especially in an aqueous solution. As a result, when porphin products are used in the field, the strict operations are required to achieve excellent effects, which reduce the convenience of product use and increases the uncertainty of product use effects. Therefore, it is necessary to develop a product with high stability, use convenience, and strong activity to meet the needs of actual use.

## SUMMARY

Objective of the present disclosure: In order to solve the problems of the prior art, the present disclosure provides a natural porphin salt and a use thereof as a plant growth regulator and a plant immunity inducer. The natural porphin salt has excellent photothermal stability, a strong activity, and a prominent control effect, can be quickly prepared into an aqueous solution, and can be easily applied to the field.

Technical solutions: To achieve the above objective, the present disclosure adopts the following technical solutions:

The present disclosure prepares a series of natural porphin salts, including a salt of a porphin compound or a salt of a chlorin compound.

The present disclosure adopts a natural porphin compound or chlorin compound. The porphin compound includes protoporphyrin and a series of protoporphyrin chelates, and the chlorin compound includes pheophorbide and a series of pheophorbide chelates.

The salt of the porphin compound in the present disclosure is prepared through a salt-producing reaction of acidic protoporphyrin or a chelate thereof with a metal ion necessary for plant nutrition. The acidic protoporphyrin and the chelate thereof include: protoporphyrin, chlorhematin (heme), and hydroxyhematin. The metal ion necessary for plant nutrition includes: a conventional monovalent, divalent, or trivalent ion of sodium, potassium, ammonium, magnesium, calcium, iron, zinc, manganese, and copper.

Protoporphyrin is a product obtained through chelated iron removal of heme extracted from animal blood, and is a natural porphin structure.

The salt of the chlorin compound in the present disclosure is prepared through a salt-producing reaction of acidic pheophorbide (chlorin) or a chelate thereof with a metal ion necessary for plant nutrition. The acidic pheophorbide and the chelate thereof include: pheophorbide (chlorin), iron chlorophyllin chloride (iron chlorin), iron hydroxychlorophyllin (iron hydroxychlorin), iron chlorophyllin (ferrous chlorin, ferrous chlorophyllin, or iron (II) chlorophyllin), zinc chlorophyllin (zinc chlorin), and copper chlorophyllin (copper chlorin). The metal ion necessary for the plant nutrition includes: a conventional monovalent, divalent, or trivalent ion of sodium, potassium, ammonium, magnesium, calcium, iron, zinc, manganese, and copper.

The chlorin, also known as pheophorbide, is a product obtained through hydrolysis-based magnesium removal of chlorophyll extracted from a plant or silkworm excrement. Chlorin is a mixture of various monomers with a chlorin parent nucleus structure, mainly including: pheophorbide a, pyropheophorbide a, chlorin e6, chlorin e4, chlorin f, chlorin P6, purpurin 18, and other monomer compounds.

The porphin salt of the present disclosure is prepared with a commercially-available chlorophyllin product such as pheophorbide (a sodium salt), sodium copper chlorophyllin, sodium iron chlorophyllin, sodium zinc chlorophyllin, iron chlorin, protoporphyrin disodium, or heme as a raw material, and a porphin salt product with a content of 95% or more (determined by spectrophotometry) can generally be obtained.

When the salt of the porphin compound or the chlorin compound in the present disclosure is a monovalent metal ion salt, the monovalent metal ion salt is prepared as follows: preparing an acidic porphin compound or an acidic chlorin compound into a first solution with an alcohol as a solvent, and mixing the first solution with an alcohol solution of a monovalent metal hydroxide for precipitation; or subjecting the acidic porphin compound or the acidic chlorin compound and the monovalent metal hydroxide to a salt-producing reaction in an aqueous solution, and conducting precipitation with acetone as a solvent; or preparing the acidic porphin compound or the acidic chlorin compound and the monovalent metal hydroxide into a second solution with acetone as a solvent, and introducing a dry ammonia gas into the second solution for precipitation; and finally conducting filtration, washing, and drying.

The monovalent metal hydroxide includes sodium hydroxide, potassium hydroxide, ammonia water, etc.

The monovalent metal ion salt of the porphin compound or the chlorin compound in the present disclosure includes: potassium protoporphyrin, ammonium protoporphyrin, sodium chlorhematin (sodium hematin, the same below), potassium chlorhematin, ammonium chlorhematin, potassium hydroxyhematin, ammonium hydroxyhematin, sodium iron chlorin (sodium iron chlorophyllin chloride, the same below), potassium iron chlorin, ammonium iron chlorin, potassium iron hydroxychlorophyllin, sodium iron hydroxychlorophyllin, ammonium iron hydroxychlorophyllin, potassium iron chlorophyllin, ammonium iron chlorophyllin, sodium chlorin (sodium pheophorbide, the same below), potassium chlorin, ammonium chlorin, ammonium zinc chlorophyllin, ammonium copper chlorophyllin, etc.

When the salt of the porphin compound or the chlorin compound in the present disclosure is a divalent or trivalent metal ion salt, the divalent or trivalent metal ion salt is conveniently prepared as follows: mixing an aqueous solution of a sodium/potassium salt of the porphin compound or the chlorin compound with an aqueous solution of a soluble divalent or trivalent metal ion salt, and conducting precipitation, filtration, washing, and drying.

The soluble divalent or trivalent metal ion salt includes a sulfate, a hydrochloride, a nitrate, etc. of a divalent or trivalent metal ion.

A divalent metal ion salt of the porphin compound or the chlorin compound in the present disclosure includes: magnesium protoporphyrin, calcium protoporphyrin, ferrous protoporphyrin, manganese protoporphyrin, zinc protoporphyrin, copper protoporphyrin, magnesium chlorhematin, calcium chlorhematin, ferrous chlorhematin, manganese chlorhematin, zinc chlorhematin, copper chlorhematin, magnesium hydroxyhematin, calcium hydroxyhematin, ferrous hydroxyhematin, manganese hydroxyhematin, zinc hydroxyhematin, copper hydroxyhematin, magnesium iron chlorin, calcium iron chlorin, ferrous iron chlorin, manganese iron chlorin, zinc iron chlorin, copper iron chlorin, magnesium iron hydroxychlorophyllin, calcium iron hydroxychlorophyllin, ferrous iron hydroxychlorophyllin, manganese iron hydroxychlorophyllin, zinc iron hydroxychlorophyllin, copper iron hydroxychlorophyllin, magnesium iron chlorophyllin, calcium iron chlorophyllin, ferrous iron chlorophyllin, manganese iron chlorophyllin, zinc iron chlorophyllin, copper iron chlorophyllin, magnesium chlorin, ferrous chlorin, manganese chlorin, zinc chlorin, copper chlorin, magnesium zinc chlorophyllin, calcium zinc chlorophyllin, ferrous zinc chlorophyllin, manganese zinc chlorophyllin, zinc zinc chlorophyllin, copper zinc chlorophyllin, magnesium copper chlorophyllin, calcium copper chlorophyllin, ferrous copper chlorophyllin, manganese copper chlorophyllin, zinc copper chlorophyllin, copper copper chlorophyllin, etc.

A trivalent metal ion salt of the porphin compound or the chlorin compound in the present disclosure includes: iron protoporphyrin, iron heme chloride, iron hydroxyhematin, iron iron chlorin (iron iron chlorophyllin chloride), iron iron hydroxychlorophyllin, iron iron chlorophyllin, iron chlorin, iron zinc chlorophyllin, iron copper chlorophyllin, etc.

The porphin salt of the present disclosure is used as a plant growth regulator or a plant immunity inducer, and the porphin salt includes:

a sodium, potassium, ammonium, magnesium, calcium, ferrous iron, ferric iron, zinc, manganese, and copper salt of protoporphyrin,

a sodium, potassium, ammonium, magnesium, calcium, ferrous iron, ferric iron, zinc, manganese, and copper salt of chlorhematin,

a sodium, potassium, ammonium, magnesium, calcium, ferrous iron, ferric iron, zinc, manganese, and copper salt of hydroxyhematin,

a sodium, potassium, ammonium, magnesium, calcium, ferrous iron, ferric iron, zinc, manganese, and copper salt of iron chlorin,

a sodium, potassium, ammonium, magnesium, calcium, ferrous iron, ferric iron, zinc, manganese, and copper salt of iron hydroxychlorophyllin,

a sodium, potassium, ammonium, magnesium, calcium, ferrous iron, ferric iron, zinc, manganese, and copper salt of iron chlorophyllin,

a sodium, potassium, ammonium, magnesium, calcium, ferrous iron, ferric iron, zinc, manganese, and copper salt of chlorin,

a sodium, potassium, ammonium, magnesium, calcium, ferrous iron, ferric iron, zinc, manganese, and copper salt of zinc chlorophyllin, and

a sodium, potassium, ammonium, magnesium, calcium, ferrous iron, ferric iron, zinc, manganese, and copper salt of copper chlorophyllin.

The porphin salt of the present disclosure retains a large ring structure of porphin and has characteristic absorption in Soret and Q bands of ultraviolet-visible spectroscopy. Due to the influence of a metal ion for salt production, a maximum absorption peak ($\lambda_{max}$) of each spectral band for the porphin salt is shifted to some degree compared with acidic porphin. The Q band in a visible region has relatively weak absorption, but still makes the porphin salt product maintain a dark color. In an aqueous solution, a difference in an absorption intensity of the Soret band (360 nm to 420 nm) of the porphin salt can be easily observed. Therefore, the absorption intensity of the Soret band can be used for the identification or verification of a salt product (UV-visible spectra of porphyrin and it's derivatives, Spectroscopy Laboratory, 28: 3, 2011, 5: 1165-1169), and is also the basis for spectrophotometric determination of a content of a porphin product (National Food Safety Standard - Food Additive - Potassium Copper Chlorophyllin: GB1886.307-2020). Compared with acidic porphin, the porphin salt undergoes a redshift of a Soret band $\lambda_{max}$ because an electron density of a porphin ring conjugated structure increases due to the electron-donating characteristic of a metal. The stronger the metallicity, the larger the redshift amplitude. For example, heme (in a 20% methanol-water solution) has $\lambda_{max}$ of 368 nm. Alkali metal and alkaline earth metal salts of the heme (in an aqueous solution) such as sodium hematin, potassium hematin, magnesium hematin, and calcium hematin all have $\lambda_{max}$ red-shifted to 393 nm to 395 nm. Transition metal salts of the heme undergo different $\lambda_{max}$ redshift amplitudes because factors such as participation of a d orbit in conjugation, ion valence, elemental electronegativity, and d electron number all can affect the change of an electron density of porphin. For example, manganese hematin has $\lambda_{max}$ of 395 nm, and undergoes the same $\lambda_{max}$ redshift amplitude as alkali metal salts, which may be related to the valence shell $d^5$ electron structure and the small electronegativity (1.5) of manganese. Zinc hematin has $\lambda_{max}$ of 377 nm, and undergoes a significantly-reduced redshift amplitude compared with manganese hematin because zinc has a valence shell $d^{10}$ electron structure and slightly-increased electronegativity (1.6) compared with manganese. Ferrous hematin (II) and ferric hematin (III) have $\lambda_{max}$ values of 371 nm and 373 nm, respectively, which correspond to the large number of electrons provided by high-valence iron. Ferrous protoporphyrin and ferric protoporphyrin have $\lambda_{max}$ values of 362 nm and 356 nm, respectively. There is no chelated metal on a protoporphyrin ring, but the ferric (III) salt undergoes a blueshift, which may be related to the fact that the valence shell $d^5$ structure is easy to accept feedback electrons to reduce an electron density of porphin conjugation. Copper hematin has $\lambda_{max}$ of 367 nm, and undergoes a blueshift, which may be related to the valence shell $d^9$ structure characteristic and the large electronegativity of 1.9 of copper.

The porphin salt of the present disclosure has an obvious dehydration-heat absorption process (88°C to 120°C) according to differential thermal analysis/thermogravimetry (DTA/TG). Thermogravimetry (TG) analysis results show that, generally, 2 moles of water are removed from a monovalent metal salt and 3 moles of water are removed from a divalent metal salt. In contrast, the acidic porphin undergoes no obvious weight loss or undergoes a significantly-smaller weight loss than a corresponding salt before decomposition, indicating that the acidic porphin does not contain water or contains very little water internally and has a compact internal structure. Differential thermal analysis (DTA) results show that the porphin salt often presents an obvious or prominent endothermic peak/exothermic peak. After dehydration, the specific heat of a system gradually increases with the increase of a temperature, but there is no phase-transition characteristic peak. The porphin salt mostly has a higher decomposition temperature than corresponding acidic porphin because of a high dehydration temperature and a large endothermic interval (the dehydration requires large energy consumption), indicating that a small amount of solid water adsorbed by the porphin salt mainly exists in the form of hydrated ions. The acidic porphin has a relatively-smooth differential thermal curve, and neither an endothermic peak nor an exothermic peak of the acidic porphin is obvious.

Among the porphin salts of the present disclosure, monovalent metal ion salts have excellent water solubility, and divalent/trivalent metal ion salts are soluble or slightly soluble in water with significantly-higher solubility than corresponding acidic porphin. At a dilute concentration (10 ppm to 0.001 ppm) for the use of the present disclosure, an aqueous solution of the porphin salt is neutral or even weakly-acidic (pH = 6.5 to 7.5) that meets the physiological requirements of plants, and has excellent photothermal stability. Accelerated tests show that the porphin salt solid of the present disclosure does not undergo a content change during storage of 2 years or more, and an aqueous solution of the porphin salt undergoes no obvious degradation. Generally, a content of an aqueous solution of the porphin salt is not significantly reduced when the aqueous solution is exposed to normal light (non-direct strong light) for 2 h to 3 h, which can meet the field needs and allow the convenient use.

Tests have proved that, when an aqueous solution of the porphin salt of the present disclosure is exposed to high-intensity direct light (illuminance: 30,000 lux) for 1 h to 3 h, a content of the aqueous solution is always significantly higher than contents of the existing acidic products such as chlorhematin and iron chlorin.

Water can be directly added to the porphin salt of the present disclosure and then stirring is conducted to easily prepare a solution with a porphin salt content of 0.01 ppm to 10 ppm (about 0.014 $\mu$mol/L to 14.0 $\mu$mol/L) that can be used as a plant growth regulator or a plant immunity inducer.

A 0.001 ppm to 0.1 ppm solution of the porphin salt of the present disclosure can be used for seed soaking and field irrigation, and a 0.01 ppm to 10.0 ppm solution of the porphin salt of the present disclosure can used for foliar spraying. The application of the porphin salt of the present disclosure can promote the seed germination, improve the germination rate, increase the root length, promote the growth of roots, enhance the immunity and stress resistance of plants, accelerate the growth of seedlings, increase the chlorophyll content, delay the premature senescence of plants, and improve the yield and a quality.

The porphin salt of the present disclosure still retains a chlorophyllase inhibition effect of acidic chlorin. An *in vitro* test for the porphin salt proves that a chlorophyllase inhibition rate of the porphin salt increases with the increase of a concentration of a salt sample, and thus the porphin salt is a typical competitive inhibitor. Tests have proved that the porphin salt of the present disclosure can also exhibit a plurality of plant signal-regulating effects, such as regulating a NO concentration of roots of a crop to promote the growth of roots, inducing the increase of activities of superoxide dismutase (SOD), catalase (CAT), and peroxidase (POD) in plant leaves under salt stress, and promoting the release of proline *in vivo*, thereby reducing the oxidative damage caused by salt stress, enhancing the stress resistance of a crop, and significantly alleviating the damage caused by herbicides to greatly increase the crop yield. *In vitro* tests for blight resistance have proved that the porphin salt of the present disclosure has an excellent plant immunity-inducing activity, can significantly weaken the infection of *Phytophthora capsici,* and has a significant control effect for blights in pepper plants artificially-inoculated with *Phytophthora* spores. When a 2.0 ppm solution of the porphin salt is sprayed, a control effect reaches 71.89%. When a 0.2 ppm solution of sodium iron chlorin is sprayed to a tobacco field for disease control, a control effect reaches 71.00%. It can be known that the porphin salt of the present disclosure can significantly improve the stress resistance of plants to increase the crop yield.

Technical effects: Compared with the prior art, the porphin salt of the present disclosure has high stability, exhibits a stable and reliable quality during long-term storage, has a long shelf life, and is easy to carry and transport. The porphin salt has prominent water solubility, is easily prepared into an aqueous solution with a stable content, and exhibits excellent reproducibility when applied to the field. The porphin salt can be conveniently applied at a low concentration with a strong activity and a prominent control effect. Raw materials for preparing the porphin salt are natural and widely available. The preparation of the porphin salt is emission-free, pollution-free, energy-saving, and eco-friendly. In particular, when used as a plant immunity inducer, the porphin salt exhibits a significantly-better control effect than the existing products.

## BRIEF DESCRIPTION OF THE DRAWINGS

FIG. 1 shows comparison of DTA/TG between sodium iron chlorophyllin and iron chlorophyllin, where the upper part is for DTA and the lower part is for TG; and
FIG. 2 shows ultraviolet spectrophotometry spectra of chlorhematin and magnesium chlorhematin solutions in a stability test under 3 h strong light.

## DETAILED DESCRIPTION OF THE EMBODIMENTS

The present disclosure is further clarified below in conjunction with specific examples.

Example 1: 5.0 g of protoporphyrin was weighed and dissolved in 1,000 mL of ethanol, and 12.0 mL of a solution of 10% potassium hydroxide in ethanol was slowly added dropwise under stirring to produce a mixed solution. The mixed solution was placed in a refrigerator overnight, filtered, washed with cold ethanol, and dried under reduced pressure to produce about 4.35 g of **potassium protoporphyrin.** The potassium protoporphyrin was a purple-brown solid, which could be decomposed at a temperature higher than 302°C (DTA/TG), was easily soluble in water, and had $\lambda_{wax}$ of 372 nm (in an aqueous solution).

Example 2: 5.0 g of protoporphyrin was weighed and dissolved in 1,000 mL of acetone, and a dry ammonia gas was slowly introduced under stirring until it was saturated to produce a mixed solution. The mixed solution was placed overnight, filtered, washed with a small amount of cold acetone, and dried under reduced pressure to produce about 3.11 g of **ammonium protoporphyrin.** The ammonium protoporphyrin was a purple-brown solid, which could be decomposed at a temperature higher than 296°C (DTA/TG), was easily soluble in water, and had $\lambda_{wax}$ of 371 nm (in an aqueous solution).

Example 3: 5.0 g of protoporphyrin disodium was weighed and dissolved in 100 mL of water under stirring to produce a first solution. 5.0 g of magnesium sulfate was weighed and dissolved in 50 mL of water under stirring to produce a second solution. The first solution and the second solution were slowly mixed under stirring, then further stirred for 1 h, placed overnight, filtered, washed with cold water, and dried under reduced pressure to produce about 3.50 g of **magnesium protoporphyrin.** The magnesium protoporphyrin was a purple-brown solid, which could be decomposed at a temperature higher than 361°C (DTA/TG), was soluble in water, and had $\lambda_{max}$ of 355 nm (in an aqueous solution).

Example 4: 5.0 g of protoporphyrin disodium was weighed and dissolved in 100 mL of water under stirring to produce a first solution. 4.5 g of calcium sulfate was weighed and dissolved in 50 mL of water under stirring to produce a second solution. The first solution and the second solution were slowly mixed under stirring, then further stirred for 1 h, placed overnight, filtered, washed with cold water, and dried under reduced pressure to produce about 3.93 g of **calcium protoporphyrin.** The calcium protoporphyrin was a purple-brown solid, which could be decomposed at a temperature higher than 365°C (DTA/TG), was soluble in water, and had $\lambda_{max}$ of 356 nm (in an aqueous solution).

Example 5: 5.0 g of protoporphyrin disodium was weighed and dissolved in 100 mL of water under stirring to produce a first solution. 6.0 g of manganese sulfate was weighed and dissolved in 50 mL of water under stirring to produce a second solution. The first solution and the second solution were slowly mixed under stirring, then further stirred for 1 h, placed overnight, filtered, washed with cold water, and dried under reduced pressure to produce about 3.83 g of **manganese protoporphyrin.** The manganese protoporphyrin was a purple-brown solid, which could be decomposed at a temperature higher than 367°C (DTA/TG), was soluble in water, and had $\lambda_{max}$ of 357 nm (in an aqueous solution).

Example 6: 5.0 g of protoporphyrin disodium was weighed and dissolved in 100 mL of water under stirring to produce a first solution. 6.5 g of ferrous sulfate was weighed and dissolved in 50 mL of water under stirring to produce a second solution. The first solution and the second solution were slowly mixed under stirring, then further stirred for 1 h, placed overnight, filtered, washed with cold water, and dried under reduced pressure to produce about 4.51 g of **ferrous protoporphyrin.** The ferrous protoporphyrin was a purple-brown solid, which could be decomposed at a temperature higher than 359°C (DTA/TG), was soluble in water, and had $\lambda_{max}$ of 362 nm (in an aqueous solution).

Example 7: 5.0 g of protoporphyrin disodium was weighed and dissolved in 100 mL of water under stirring to produce a first solution. 4.5 g of ferric chloride was weighed and dissolved in 50 mL of water under stirring to produce a second solution. The first solution and the second solution were slowly mixed under stirring, then further stirred for 1 h, placed for 1 h, filtered, washed with cold water, and dried under reduced pressure to produce about 4.63 g of **ferric protoporphyrin.** The ferric protoporphyrin was a purple-brown solid, which could be decomposed at a temperature higher than 367°C (DTA/TG), was slightly soluble in water, and had $\lambda_{wax}$ of 356 nm (in an aqueous solution).

Example 8: 5.0 g of protoporphyrin disodium was weighed and dissolved in 100 mL of water under stirring to produce a first solution. 5.0 g of zinc sulfate was weighed and dissolved in 50 mL of water under stirring to produce a second solution. The first solution and the second solution were slowly mixed under stirring, then further stirred for 1 h, placed overnight, filtered, washed with cold water, and dried under reduced pressure to produce about 4.23 g of **zinc protoporphyrin.** The zinc protoporphyrin was a purple-brown solid, which could be decomposed at a temperature higher than 361°C (DTA/TG), was slightly soluble in water, and had $\lambda_{wax}$ of 358 nm (in an aqueous solution).

Example 9: 5.0 g of protoporphyrin disodium was weighed and dissolved in 100 mL of water under stirring to produce a first solution. 6.0 g of copper sulfate was weighed and dissolved in 50 mL of water under stirring to produce a second solution. The first solution and the second solution were slowly mixed under stirring, then further stirred for 1 h, placed overnight, filtered, washed with cold water, and dried under reduced pressure to produce about 4.72 g of **copper protoporphyrin.** The copper protoporphyrin was a purple-brown solid, which could be decomposed at a temperature higher than 363°C (DTA/TG), was slightly soluble in water, and had $\lambda_{wax}$ of 382 nm (in an aqueous solution).

Example 10: 10.0 g of chlorhematin was weighed and dissolved in 1,000 mL of ethanol, and 25.0 mL of a solution of 10% potassium hydroxide in ethanol was slowly added dropwise under stirring to produce a mixed solution. The mixed solution was placed overnight, filtered, washed with cold ethanol, and dried under reduced pressure to produce about 7.14 g of **potassium chlorhematin.** The potassium chlorhematin was a black solid powder, which could be decomposed at a temperature higher than 251°C (DTA/TG), was easily soluble in water, and had $\lambda_{max}$ of 394 nm (in an aqueous solution).

Example 11: 10.0 g of chlorhematin was weighed and dissolved in 1,000 mL of ethanol, and 25.0 mL of a solution of 10% sodium hydroxide in ethanol was slowly added dropwise under stirring to produce a mixed solution. The mixed solution was placed overnight, filtered, washed with cold ethanol, and dried under reduced pressure to produce about 9.33 g of **sodium chlorhematin.** The sodium chlorhematin was a black solid powder, which could be decomposed at a temperature higher than 264°C (DTA/TG), was easily soluble in water, and had $\lambda_{max}$ of 393 nm (in an aqueous solution).

Comparative sample: Preparation of sodium hydroxyhematin

According to the method in the literature (Preparation of Sodium Chlorhematin, Chemistry World, 2004.10: 540-541): 10.0 g of chlorhematin was dissolved in 120 mL of a 0.2 mol/L NaOH solution to produce a mixed solution, and the mixed solution was stirred for 6 h and filtered to produce a filtrate. 1,000 mL of acetone (8 times or more an amount of the filtrate) was slowly added to the filtrate for precipitating a black precipitate to produce a mixture, and the mixture was placed overnight, filtered, washed with a small amount of acetone, and dried under reduced pressure to produce 8.7 g of a black solid. The black solid was easily soluble in water, and had $\lambda_{max}$ of 384 nm (in an aqueous solution).

Comparative test 1: Detection of Cl⁻ ions

Reagents: A HNO$_3$ solution (4 mol/L) and a AgNO$_3$ solution (0.1 mol/L).

Method: A filtrate (reaction mother liquor) produced after the filtration of a precipitated product in the above preparation was collected, subjected to rotary evaporation to completely recover the solvent ethanol or acetone, and cooled to obtain a residual solid. The residual solid was dissolved with about 20 mL of pure water and then adjusted with HNO$_3$ to neutrality (a pH was about 7) to produce a solution to be tested. A small amount of the solution to be tested (0.5 mL to 1 mL) was taken and added to a test tube, few drops of the AgNO$_3$ solution were added dropwise, and whether the precipitation/turbidity occurred was observed. If the turbidity occurred, a small amount of HNO$_3$ was further added slowly, and a change was observed.

Results: A solution to be tested obtained after ethanol was recovered from a filtrate did not turned turbid, indicating that no Cl⁻ ion was detected.

A solution to be tested obtained after acetone was recovered from a filtrate underwent precipitation (AgCl↓), and a precipitate did not change after HNO$_3$ was added, indicating that Cl⁻ ions were detected.

It indicated that a product prepared in ethanol (in this example) was sodium chlorhematin. In the process of adding acetone to a chlorhematin alkaline solution for precipitation to prepare the comparative sample (the method in the literature), a chloride ion was replaced with hydroxyl to enter a reaction solution (NaCl was generated), and a resulting product was chlorine-free (replaced with hydroxyl) and should be sodium hydroxyhematin rather than sodium chlorhematin.

Comparative test 2: Determination of a maximum ultraviolet absorption wavelength ($\lambda_{max}$) for samples

Samples: Sodium chlorhematin, sodium hydroxyhematin, and chlorhematin.

Reagents: Pure water and a 0.1 mol/L NaOH solution.

Instrument: Shimadzu UV2600 ultraviolet spectrophotometer.

Method: An appropriate amount of a sample was weighed, dissolved with pure water or a NaOH solution, and then diluted into a solution with a concentration of about 20.0 ppm. A maximum absorption wavelength $\lambda_{max}$ (structural characteristic) of the sample was determined by an ultraviolet scanner. Results were shown in the following table:

Maximum ultraviolet absorption wavelengths ($\lambda_{max}$) of the samples in different solvents

| Sample | $\lambda_{max}$ (nm) | |
| --- | --- | --- |
| | Pure water | NaOH solution |
| Sodium chlorhematin | 393 | 384 |
| Sodium hydroxyhematin | 384 | 384 |
| Chlorhematin | -- | 384 |

Results: Characteristic absorption wavelengths of the three samples in the NaOH solution all were 384 nm, indicating that an axial chlorine group binding to central iron in chlorhematin was replaced by hydroxyl at a high OH$^-$ ion concentration to produce a product sodium hydroxyhematin. In a solution prepared with an aqueous solution, a OH$^-$ ion concentration was not high (a 20 ppm sodium chlorhematin solution with a pH of 7.5), and a chlorine group was retained. The sample in this example had a characteristic absorption wavelength of 393 nm for sodium chlorhematin. The comparative sample prepared by the method in the literature had exactly the same characteristic value as the NaOH solution, indicating that the sample was sodium hydroxyhematin.

The axial coordination groups chlorine and hydroxyl for central iron in heme have different electron-donating/-withdrawing abilities, and the action results are related to the electronegativity, lone electron pairs, and valence-shell electron structures of elements. Chlorine has electronegativity of 3.0 and a 3p valence-shell structure, and 5 pairs of lone electrons can be used when chlorine axially binds to central iron. Oxygen in hydroxyl has electronegativity of 3.5 and a 2p valence-shell structure, and 4 pairs of lone electrons can be used when oxygen axially binds to central iron. It can be seen that hydroxyl has a stronger electron-withdrawing ability and a weaker electron-feedback ability than chlorine. Therefore, the hydroxyl coordination leads to the decrease in an electron density of a porphin conjugation structure, and makes a characteristic absorption Soret band blue-shifted from 393 nm to 384 nm of sodium hydroxyhematin.

Example 12: 5.0 g of chlorhematin was weighed and dissolved in 1,000 mL of acetone, and a dry ammonia gas was slowly introduced under stirring until it was saturated to produce a mixed solution. The mixed solution was placed overnight, filtered, washed with a small amount of cold acetone, and dried under reduced pressure to produce about 3.60 g of ammonium chlorhematin. The ammonium chlorhematin was a brown solid, which could be decomposed at a temperature higher than 243°C (DTA/TG), was easily soluble in water, and had $\lambda_{max}$ of 386 nm (in an aqueous solution).

Example 13: 10.0 g of sodium chlorhematin was weighed and dissolved in 200 mL of water under stirring to produce a first solution. 11.0 g of magnesium sulfate was weighed and dissolved in 100 mL of water under stirring to produce a second solution. The first solution and the second solution were slowly mixed under stirring, then further stirred for 1 h, placed overnight, filtered, washed with cold water, and dried under reduced pressure to produce about 7.19 g of **magnesium chlorhematin.** The magnesium chlorhematin was a dark-brown solid, which could be decomposed at a temperature higher than 292°C (DTA/TG), was soluble in water, and had $\lambda_{max}$ of 395 nm (in an aqueous solution).

Example 14: 10.0 g of sodium chlorhematin was weighed and dissolved in 200 mL of water under stirring to produce a first solution. 9.0 g of calcium chloride was weighed and dissolved in 100 mL of water under stirring to produce a second solution. The first solution and the second solution were slowly mixed under stirring, then further stirred for 1 h, placed overnight, filtered, washed with cold water, and dried under reduced pressure to produce about 7.37 g of **calcium chlorhematin.** The calcium chlorhematin was a dark-brown solid, which could be decomposed at a temperature higher than 311°C (DTA/TG), was soluble in water, and had $\lambda_{wax}$ of 395 nm (in an aqueous solution).

Example 15: 10.0 g of sodium chlorhematin was weighed and dissolved in 200 mL of water under stirring to produce a first solution. 11.0 g of manganese sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 8.57 g of **manganese chlorhematin.** The manganese chlorhematin was a dark-brown solid, which could be decomposed at a temperature higher than 329°C (DTA/TG), was slightly soluble in water, and had $\lambda_{max}$ of 395 nm (in an aqueous solution).

Example 16: 10.0 g of sodium chlorhematin was weighed and dissolved in 200 mL of water under stirring to produce a first solution. 14.0 g of ferrous sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 8.63 g of **ferrous chlorhematin.** The ferrous chlorhematin was a dark-brown solid, which could be decomposed at a temperature higher than 327°C (DTA/TG), was slightly soluble in water, and had $\lambda_{wax}$ of 373 nm (in an aqueous solution).

Example 17: 10.0 g of sodium chlorhematin was weighed and dissolved in 200 mL of water under stirring to produce a first solution. 11.0 g of ferric chloride was weighed and dissolved in 120 mL of water under stirring to produce a second solution. The first solution and the second solution were slowly mixed under stirring, then further stirred for 1 h, placed overnight, filtered, washed with cold water, and dried under reduced pressure to produce about 9.06 g of **ferric chlorhematin.** The ferric chlorhematin was a dark-brown solid, which could be decomposed at a temperature higher than 334°C (DTA/TG), was slightly soluble in water, and had $\lambda_{wax}$ of 371 nm (in an aqueous solution).

Example 18: 10.0 g of sodium chlorhematin was weighed and dissolved in 200 mL of water under stirring to produce a first solution. 14.0 g of zinc sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 9.13 g of **zinc chlorhematin.** The zinc chlorhematin was a dark-brown solid, which could be decomposed at a temperature higher than 274°C (DTA/TG), was slightly soluble in water, and had $\lambda_{max}$ of 377 nm (in an aqueous solution).

Example 19: 10.0 g of sodium chlorhematin was weighed and dissolved in 200 mL of water under stirring to produce a first solution. 15.0 g of copper sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 9.06 g of **copper chlorhematin.** The copper chlorhematin was a dark-brown solid, which could be decomposed at a temperature higher than 248°C (DTA/TG), was slightly soluble in water, and had $\lambda_{max}$ of 367 nm (in an aqueous solution).

Example 20: 1,000.0 g of technical-grade iron chlorin (produced by Nanjing Baite Bioengineering Co., Ltd., the same below) was weighed and mixed with 15 L of an industrial alcohol, and stirring was conducted to allow full dissolution. 2.5 L of a solution of 15% potassium hydroxide in ethanol was slowly added dropwise to produce a precipitate. The precipitate was placed overnight, separated through filtration, washed with cold ethanol, and dried under reduced pressure to produce 896.0 g of **potassium iron chlorin.** The potassium iron chlorin was a dark-green powder, which could be decomposed at a temperature higher than 313°C (DTA/TG), was easily soluble in water, and had $\lambda_{wax}$ of 400 nm (in an aqueous solution).

Example 21: 1,000.0 g of technical-grade iron chlorin was weighed and mixed with 15 L of an industrial alcohol, and full dissolution was allowed. 2.3 L of a solution of 15% sodium hydroxide in ethanol was slowly added dropwise to produce a precipitate. The precipitate was placed overnight, separated through filtration, washed with cold ethanol, and dried under reduced pressure to produce 907 g of **sodium iron chlorin.** The sodium iron chlorin was a dark-green powder, which could be decomposed at a temperature higher than 301°C (DTA/TG), was easily soluble in water, and had $\lambda_{max}$ of 399 nm (in an aqueous solution).

Example 22: 100.0 g of technical-grade iron chlorin was weighed and mixed with 1.5 L of acetone, and dissolution was allowed. A dry ammonia gas was slowly introduced to produce a precipitate. The precipitate was placed overnight, separated through filtration, washed with cold acetone, and dried under reduced pressure to produce about 51.2 g of **ammonium iron chlorin.** The ammonium iron chlorin was a dark-green powder, which could be decomposed at a temperature higher than 270°C (DTA/TG), was easily soluble in water, and had $\lambda_{wax}$ of 401 nm (in an aqueous solution).

Example 23: 100.0 g of sodium iron chlorin was weighed and dissolved in 1 L of water under stirring to produce a first solution. 110.0 g of magnesium chloride hexahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed 2 times with cold water, separated through pressure filtration, and then dried under reduced pressure to produce 73.1 g of **magnesium iron chlorin.** The magnesium iron chlorin was a dark-green powder, which could be decomposed at a temperature higher than 302°C (DTA/TG), was soluble in water, and had $\lambda_{wax}$ of 400 nm (in an aqueous solution).

Example 24: 100.0 g of sodium iron chlorin was weighed and dissolved in 1 L of water under stirring to produce a first solution. 100.0 g of calcium chloride dihydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 80.2 g of **calcium iron chlorin.** The calcium iron chlorin was a dark-green powder, which could be decomposed at a temperature higher than 377°C (DTA/TG), was soluble in water, and had $\lambda_{wax}$ of 399 nm (in an aqueous solution).

Example 25: 100.0 g of sodium iron chlorin was weighed and dissolved in 1 L of water under stirring to produce a first solution. 110.0 g of ferrous chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 91.1 g of **ferrous iron**

**chlorin.** The ferrous iron chlorin was a dark-green powder, which could be decomposed at a temperature higher than 303°C (DTA/TG), was slightly soluble in water, and had $\lambda_{wax}$ of 401 nm (in an aqueous solution).

Example 26: 100.0 g of sodium iron chlorin was weighed and dissolved in 1 L of water under stirring to produce a first solution. 110.0 g of ferric chloride hexahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 89.2 g of **ferric iron chlorin.** The ferric iron chlorin was a dark-green powder, which could be decomposed at a temperature higher than 329°C (DTA/TG), was slightly soluble in water, and had $\lambda_{wax}$ of 405 nm (in an aqueous solution).

Example 27: 100.0 g of sodium iron chlorin was weighed and dissolved in 1 L of water under stirring to produce a first solution. 100.0 g of manganese chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 87.8 g of **manganese iron chlorin.** The manganese iron chlorin was a dark-green powder, which could be decomposed at a temperature higher than 299°C (DTA/TG), was slightly soluble in water, and had $\lambda_{wax}$ of 400 nm (in an aqueous solution).

Example 28: 100.0 g of sodium iron chlorin was weighed and dissolved in 1 L of water under stirring to produce a first solution. 100.0 g of zinc chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 91.4 g of **zinc iron chlorin.** The zinc iron chlorin was a dark-green powder, which could be decomposed at a temperature higher than 300°C (DTA/TG), was slightly soluble in water, and had $\lambda_{max}$ of 399 nm (in an aqueous solution).

Example 29: 100.0 g of sodium iron chlorin was weighed and dissolved in 1 L of water under stirring to produce a first solution. 95.0 g of copper chloride dihydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 87.2 g of **copper iron chlorin.** The copper iron chlorin was a dark-green powder, which could be decomposed at a temperature higher than 222°C (DTA/TG), was slightly soluble in water, and had $\lambda_{max}$ of 398 nm (in an aqueous solution).

Example 30: 1,000.0 g of commercially-available sodium iron chlorophyllin was weighed and dissolved in 10 L of water under stirring, and 15% dilute sulfuric acid was slowly added dropwise under stirring at room temperature until a pH was 3 to 4 to produce a mixed solution. The mixed solution was placed in the dark overnight and filtered to produce a filter cake. The filter cake was washed 2 times with cold water, separated through pressure filtration, and then dried under reduced pressure to produce iron chlorophyllin. The iron chlorophyllin was mixed with 15 L of an industrial alcohol, and stirring was conducted for dissolution. 2.5 L of a solution of 15% potassium hydroxide in ethanol was slowly added dropwise to produce a precipitate. The precipitate was placed overnight, separated through filtration, washed with cold ethanol, and dried under reduced pressure to produce about 783.0 g of **potassium iron chlorophyllin.** The potassium iron chlorophyllin was a dark-green powder, which could be decomposed at a temperature higher than 309°C (DTA/TG), was easily soluble in water, and had $\lambda_{max}$ of 397 nm (in an aqueous solution).

Example 31: 100.0 g of commercially-available sodium iron chlorophyllin was weighed and dissolved in 1 L of water under stirring, and 15% dilute sulfuric acid was slowly added dropwise under stirring at room temperature until a pH was 3 to 4 to produce a mixed solution. The mixed solution was placed in the dark overnight and filtered to produce a filter cake. The filter cake was washed 2 times with cold water, separated through pressure filtration, and then dried under reduced pressure to produce iron chlorophyllin. The iron chlorophyllin was mixed with 1.5 L of acetone, and dissolution was allowed. A dry ammonia gas was slowly introduced under stirring to produce a precipitate. The precipitate was placed overnight, separated through filtration, washed with cold acetone, and dried under reduced pressure to produce about 37.2 g of **ammonium iron chlorophyllin.** The ammonium iron chlorophyllin was a dark-green powder, which could be decomposed at a temperature higher than 224°C (DTA/TG), was easily soluble in water, and had $\lambda_{max}$ of 398 nm (in an aqueous solution).

Example 32: 100.0 g of commercially-available sodium iron chlorophyllin was weighed and dissolved in 1 L of water under stirring to produce a first solution. 120.0 g of ferrous sulfate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 90.9 g of **ferrous iron chlorophyllin.** The ferrous iron chlorophyllin was a dark-green powder, which could be decomposed at a temperature higher than 278°C (DTA/TG), was slightly soluble in water, and had $\lambda_{max}$ of 400 nm (in an aqueous solution).

Example 33: 100.0 g of commercially-available sodium iron chlorophyllin was weighed and dissolved in 1 L of water under stirring to produce a first solution. 90.0 g of ferric chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 91.6 g of **ferric iron chlorophyllin.** The ferric iron chlorophyllin was a dark-green powder, which could be decomposed at a temperature higher than 343°C (DTA/TG), was slightly soluble in water, and had $\lambda_{wax}$ of 399 nm (in an aqueous solution).

Example 34: 100.0 g of commercially-available sodium iron chlorophyllin was weighed and dissolved in 1 L of water under stirring to produce a first solution. 110.0 g of copper sulfate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 91.2 g of **copper iron chlorophyllin.** The copper iron chlorophyllin was a dark-green powder, which could be decomposed at a temperature higher than 208°C (DTA/TG), was slightly soluble in water, and had $\lambda_{wax}$ of 401 nm (in an aqueous solution).

Example 35: 500.0 g of commercially-available sodium zinc chlorophyllin was weighed and dissolved in 5 L of purified water, and dilute sulfuric acid was added under stirring for adjusting a pH to 4 to 5 to produce a first precipitate. The first precipitate was separated through filtration, washed with water, and oven-dried to produce zinc chlorophyllin. 100.0 g of the zinc chlorophyllin was weighed and mixed with 1.5 L of acetone, and dissolution was allowed. A dry ammonia gas was slowly introduced to produce a second precipitate. The second precipitate was placed overnight, separated through filtration, washed with cold acetone, and dried under reduced pressure to produce about 40.2 g of **ammonium zinc chlorophyllin.** The ammonium zinc chlorophyllin was a black powder, which could be decomposed at a temperature higher than 251°C (DTA/TG), was easily soluble in water, and had $\lambda_{wax}$ of 404 nm (in an aqueous solution).

Example 36: 100.0 g of commercially-available sodium zinc chlorophyllin was weighed and dissolved in 1 L of water under stirring to produce a first solution. 100.0 g of magnesium chloride hexahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed 2 times with cold water, separated through pressure filtration, and then dried under reduced pressure to produce 87.6 g of commercially-available **magnesium zinc chlorophyllin.** The magnesium zinc chlorophyllin was a black powder, which could be decomposed at a temperature higher than 333°C (DTA/TG), was soluble in water, and had $\lambda_{wax}$ of 405 nm (in an aqueous solution).

Example 37: 100.0 g of commercially-available sodium zinc chlorophyllin was weighed and dissolved in 1 L of water under stirring to produce a first solution. 100.0 g of calcium chloride dihydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 83.7 g of **calcium zinc chlorophyllin.** The calcium zinc chlorophyllin was a black powder, which could be decomposed at a temperature higher than 365°C (DTA/TG), was soluble in water, and had $\lambda_{wax}$ of 405 nm (in an aqueous solution).

Example 38: 100.0 g of commercially-available sodium zinc chlorophyllin was weighed and dissolved in 1 L of water under stirring to produce a first solution. 120.0 g of ferrous chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 90.2 g of **ferrous zinc chlorophyllin.** The ferrous zinc chlorophyllin was a black powder, which could be decomposed at a temperature higher than 371°C (DTA/TG), was slightly soluble in water, and had $\lambda_{wax}$ of 406 nm (in an aqueous solution).

Example 39: 100.0 g of commercially-available sodium zinc chlorophyllin was weighed and dissolved in 1 L of water under stirring to produce a first solution. 100.0 g of ferric chloride hexahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 89.8 g of **ferric zinc chlorophyllin.** The ferric zinc chlorophyllin was a black powder, which could be decomposed at a temperature higher than 341°C (DTA/TG), was slightly soluble in water, and had $\lambda_{wax}$ of 403 nm (in an aqueous solution).

Example 40: 100.0 g of commercially-available sodium zinc chlorophyllin was weighed and dissolved in 1 L of water under stirring to produce a first solution. 100.0 g of manganese chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 90.7 g of **manganese zinc chlorophyllin.** The manganese zinc chlorophyllin was a black powder, which could be decomposed at a temperature higher than 336°C (DTA/TG), was slightly soluble in water, and had $\lambda_{wax}$ of 404 nm (in an aqueous solution).

Example 41: 100.0 g of commercially-available sodium zinc chlorophyllin was weighed and dissolved in 1 L of water under stirring to produce a first solution. 100.0 g of zinc chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 92.5 g of **zinc zinc chlorophyllin.** The zinc zinc chlorophyllin was a black powder, which could be decomposed at a temperature higher than 320°C (DTA/TG), was slightly soluble in water, and had $\lambda_{wax}$ of 409 nm (in an aqueous solution).

Example 42: 100.0 g of commercially-available sodium zinc chlorophyllin was weighed and dissolved in 1 L of water under stirring to produce a first solution. 95.0 g of copper chloride dihydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 90.3 g of **copper zinc chlorophyllin.** The copper zinc chlorophyllin was a black powder, which could be decomposed at a temperature higher than 271°C (DTA/TG), was slightly soluble in water, and had $\lambda_{wax}$ of 409 nm (in an aqueous solution).

Example 43: 100.0 g of commercially-available sodium copper chlorophyllin was weighed and dissolved in 2 L of water under stirring, and 15% dilute sulfuric acid was slowly added dropwise under stirring at room temperature until a pH was 3 to 5 to produce a mixed solution. The mixed solution was placed in the dark overnight and filtered to produce a filter cake. The filter cake was washed 2 times with cold water, separated through pressure filtration, and then dried under reduced pressure to produce copper chlorophyllin. The copper chlorophyllin was mixed with 2 L of an industrial alcohol, and stirring was conducted for dissolution. 250 mL of a solution of 15% potassium hydroxide in ethanol was slowly added dropwise to produce a precipitate. The precipitate was placed overnight, separated through filtration, washed with cold ethanol, and dried under reduced pressure to produce about 79.8 g of **potassium copper chlorophyllin.** The potassium copper chlorophyllin was a dark-green powder, which could be decomposed at a temperature higher than 296°C (DTA/TG), was easily soluble in water, and had $\lambda_{max}$ of 405 nm (in an aqueous solution).

Example 44: 100.0 g of commercially-available sodium copper chlorophyllin was weighed and dissolved in 2 L of water under stirring, and 15% dilute sulfuric acid was slowly added dropwise under stirring at room temperature until a pH was 3 to 5 to produce a mixed solution. The mixed solution was placed in the dark overnight and filtered to produce a filter cake. The filter cake was washed 2 times with cold water, separated through pressure filtration, and then dried under reduced pressure to produce copper chlorophyllin. The copper chlorophyllin was mixed with 2 L of acetone, and stirring was conducted for dissolution. A dry ammonia gas was slowly introduced to produce a precipitate until it was saturated. The precipitate was placed overnight, separated through filtration, washed with cold acetone, and dried under reduced pressure to produce about 44.6 g of **ammonium copper chlorophyllin.** The ammonium copper chlorophyllin was a dark-green powder, which could be decomposed at a temperature higher than 277°C (DTA/TG), was easily soluble in water, and had $\lambda_{max}$ of 404 nm (in an aqueous solution).

Example 45: 100.0 g of commercially-available sodium copper chlorophyllin was weighed and dissolved in 1 L of water under stirring to produce a first solution. 100.0 g of magnesium sulfate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 74.1 g of **magnesium copper chlorophyllin.** The magnesium copper chlorophyllin was a dark-green powder, which could be decomposed at a temperature higher than 280°C (DTA/TG), was soluble in water, and had $\lambda_{max}$ of 406 nm (in an aqueous solution).

Example 46: 100.0 g of commercially-available sodium copper chlorophyllin was weighed and dissolved in 1 L of water under stirring to produce a first solution. 90.0 g of calcium chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 87.4 g of **calcium copper chlorophyllin.** The calcium copper chlorophyllin was a dark-green powder, which could be decomposed at a temperature higher than 305°C (DTA/TG), was soluble in water, and had $\lambda_{max}$ of 405 nm (in an aqueous solution).

Example 47: 100.0 g of commercially-available sodium copper chlorophyllin was weighed and dissolved in 1 L of water under stirring to produce a first solution. 90.0 g of manganese chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 70.3 g of **manganese copper chlorophyllin.** The manganese copper chlorophyllin was a dark-green powder, which could be decomposed at a temperature higher than 311°C (DTA/TG), was soluble in water, and had $\lambda_{max}$ of 405 nm (in an aqueous solution).

Example 48: 100.0 g of commercially-available sodium copper chlorophyllin was weighed and dissolved in 1 L of water under stirring to produce a first solution. 120.0 g of ferrous sulfate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 90.4 g of **ferrous copper chlorophyllin.** The ferrous copper chlorophyllin was a dark-green powder, which could be decomposed at a temperature higher than 266°C (DTA/TG), was slightly soluble in water, and had $\lambda_{wax}$ of 403 nm (in an aqueous solution).

Example 49: 100.0 g of commercially-available sodium copper chlorophyllin was weighed and dissolved in 1 L of water under stirring to produce a first solution. 90.0 g of ferric chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 89.7 g of **ferric copper chlorophyllin.** The ferric copper chlorophyllin was a dark-green powder, which could be decomposed at a temperature higher than 357°C (DTA/TG), was slightly soluble in water, and had $\lambda_{wax}$ of 406 nm (in an aqueous solution).

Example 50: 100.0 g of commercially-available sodium copper chlorophyllin was weighed and dissolved in 1 L of water under stirring to produce a first solution. 100.0 g of zinc chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 90.5 g of **zinc copper chlorophyllin.** The zinc copper chlorophyllin was a dark-green powder, which could be decomposed at a temperature higher than 263°C (DTA/TG), was slightly soluble in water, and had $\lambda_{wax}$ of 404 nm (in an aqueous solution).

Example 51: 100.0 g of commercially-available sodium copper chlorophyllin was weighed and dissolved in 1 L of water under stirring to produce a first solution. 110.0 g of copper sulfate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 91.7 g of **copper copper chlorophyllin.** The copper copper chlorophyllin was a dark-green powder, which could be decomposed at a temperature higher than 242°C (DTA/TG), was slightly soluble in water, and had $\lambda_{wax}$ of 404 nm (in an aqueous solution).

Example 52: 1,000 g of commercially-available sodium magnesium chlorophyllin was weighed and dissolved in 20 L of purified water preheated to 60°C, the heating was stopped, and dilute sulfuric acid was slowly added dropwise until a pH was 3 to 4 to produce a mixed solution. The mixed solution was placed in the dark overnight and filtered to produce a filter cake. The filter cake was washed 2 times with cold water, separated through pressure filtration, and then dried under reduced pressure to produce 886.3 g of pheophorbide (chlorin). 100.0 g of the dried chlorin was dissolved in 1.6 L of an industrial alcohol, and stirring was conducted for full dissolution. 300 mL of a solution of 15% potassium hydroxide in ethanol was slowly added dropwise to produce a precipitate. The precipitate was placed overnight, separated through filtration, washed with cold ethanol, and dried under reduced pressure to produce 69.2 g of **potassium chlorin.** The potassium chlorin was a black powder, which could be decomposed at a temperature higher than 283°C (DTA/TG), was easily soluble in water, and had $\lambda_{wax}$ of 405 nm (in an aqueous solution).

Example 53: 1,000 g of dry chlorin (which was prepared according to the method in Example 52) was weighed and mixed with 15 L of an industrial alcohol, and full dissolution was allowed. 2.3 L of a solution of 15% sodium hydroxide in ethanol was slowly added dropwise to produce a precipitate. The precipitate was placed overnight, separated through filtration, washed with cold ethanol, and dried under reduced pressure to produce 723.0 g of **sodium chlorin.** The sodium chlorin was a black powder, which could be decomposed at a temperature higher than 277°C (DTA/TG), was easily soluble in water, and had $\lambda_{max}$ of 404 nm (in an aqueous solution).

Example 54: 100.0 g of dry chlorin (which was prepared according to the method in Example 52) was weighed and mixed with 1.5 L of acetone, and dissolution was allowed. A dry ammonia gas was slowly introduced to produce a precipitate. The precipitate was placed overnight, separated through filtration, washed with cold acetone, and dried under reduced pressure to produce about 37.6 g of **ammonium chlorin.** The ammonium chlorin was a brown powder, which could be decomposed at a temperature higher than 281°C (DTA/TG), was easily soluble in water, and had $\lambda_{max}$ of 403 nm (in an aqueous solution).

Example 55: 100.0 g of sodium chlorin (which was prepared according to the method in Example 53) was weighed and dissolved in 1 L of water under stirring to produce a first solution. 110.0 g of magnesium chloride hexahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed 2 times with cold water, separated through pressure filtration, and then dried under reduced pressure to produce 80.7 g of **magnesium chlorin.** The magnesium chlorin was a dark-brown powder, which could be decomposed at a temperature higher than 312°C (DTA/TG), was soluble in water, and had

$\lambda_{max}$ of 404 nm (in an aqueous solution).

Example 56: 100.0 g of sodium chlorin (which was prepared according to the method in Example 53) was weighed and dissolved in 1 L of water under stirring to produce a first solution. 100.0 g of calcium chloride dihydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 83.1 g of **calcium chlorin.** The calcium chlorin was a black powder, which could be decomposed at a temperature higher than 373°C (DTA/TG), was soluble in water, and had $\lambda_{max}$ of 404 nm (in an aqueous solution).

Example 57: 100.0 g of sodium chlorin (which was prepared according to the method in Example 53) was weighed and dissolved in 1 L of water under stirring to produce a first solution. 110.0 g of ferrous chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 83.9 g of **ferrous chlorin.** The ferrous chlorin was a black powder, which could be decomposed at a temperature higher than 292°C (DTA/TG), was slightly soluble in water, and had $\lambda_{max}$ of 405 nm (in an aqueous solution).

Example 58: 100.0 g of sodium chlorin (which was prepared according to the method in Example 53) was weighed and dissolved in 1 L of water under stirring to produce a first solution. 110.0 g of ferric chloride hexahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 84.5 g of **ferric chlorin.** The ferric chlorin was a black powder, which could be decomposed at a temperature higher than 303°C (DTA/TG), was slightly soluble in water, and had $\lambda_{max}$ of 403 nm (in an aqueous solution).

Example 59: 100.0 g of sodium chlorin (which was prepared according to the method in Example 53) was weighed and dissolved in 1 L of water under stirring to produce a first solution. 100.0 g of manganese chloride tetrahydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 81.4 g of **manganese chlorin.** The manganese chlorin was a black powder, which could be decomposed at a temperature higher than 284°C (DTA/TG), was slightly soluble in water, and had $\lambda_{max}$ of 404 nm (in an aqueous solution).

Example 60: 100.0 g of sodium chlorin (which was prepared according to the method in Example 53) was weighed and dissolved in 1 L of water under stirring to produce a first solution. 100.0 g of zinc chloride was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 86.0 g of **zinc chlorin.** The zinc chlorin was a black powder, which could be decomposed at a temperature higher than 349°C (DTA/TG), was slightly soluble in water, and had $\lambda_{max}$ of 409 nm (in an aqueous solution).

Example 61: 100.0 g of sodium chlorin (which was prepared according to the method in Example 53) was weighed and dissolved in 1 L of water under stirring to produce a first solution. 95.0 g of copper chloride dihydrate was weighed and dissolved in 500 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 84.6 g of **copper chlorin.** The copper chlorin was a black powder, which could be decomposed at a temperature higher than 245°C (DTA/TG), was slightly soluble in water, and had $\lambda_{max}$ of 405 nm (in an aqueous solution).

Example 62: 10.0 g of chlorhematin was weighed and dissolved in 1,000 mL of a solution of 6.0% potassium hydroxide in water, and stirring was conducted in the dark for 5 h. 8,000 mL of acetone was slowly added, and stirring was conducted in the dark for 1 h to produce a mixed solution. The mixed solution was placed overnight, filtered, washed with cold acetone, and dried under reduced pressure to produce about 8.37 g of **potassium hydroxyhematin.** The potassium hydroxyhematin was a black solid powder, which could be decomposed at a temperature higher than 303°C (DTA/TG), was easily soluble in water, and had $\lambda_{max}$ of 388 nm (in an aqueous solution).

Example 63: 5.0 g of potassium hydroxyhematin was weighed and dissolved in 1,000 mL of water, and 5% sulfuric acid was slowly added dropwise under stirring in the dark until a pH was about 4 to produce a first mixed solution. The first mixed solution was placed overnight and filtered to produce a filter cake. The filter cake was washed with water until it was neutral and dried under reduced pressure to produce a solid. The solid was dissolved in 1,000 mL of acetone, and a dry ammonia gas was slowly introduced under stirring until it was saturated to produce a second mixed solution. The second mixed solution was placed overnight, filtered, washed with a small amount of cold acetone, and dried under reduced pressure to produce about 3.17 g of **ammonium hydroxyhematin.** The ammonium hydroxyhematin was a brown solid, which could be decomposed at a temperature higher than 287°C (DTA/TG), was easily soluble in water, and had $\lambda_{max}$ of 383 nm (in an aqueous solution).

Example 64: 10.0 g of potassium hydroxyhematin was weighed and dissolved in 200 mL of water under stirring to produce a first solution. 12.0 g of magnesium sulfate was weighed and dissolved in 100 mL of water under stirring to produce a second solution. The first solution and the second solution were slowly mixed under stirring, then further stirred for 1 h, placed overnight, filtered, washed with a small amount of cold water, and dried under reduced pressure to produce about 7.25 g of **magnesium hydroxyhematin.** The magnesium hydroxyhematin was a dark-brown solid, which could be decomposed at a temperature higher than 293°C (DTA/TG), was soluble in water, and had $\lambda_{max}$ of 387 nm (in an aqueous solution).

Example 65: 10.0 g of potassium hydroxyhematin was weighed and dissolved in 200 mL of water under stirring to produce a first solution. 10.0 g of calcium chloride was weighed and dissolved in 100 mL of water under stirring to produce a second solution. The first solution and the second solution were slowly mixed under stirring, then further stirred for 1 h, placed overnight, filtered, washed with cold water, and dried under reduced pressure to produce about 7.46 g of **calcium hydroxyhematin.** The calcium hydroxyhematin was a dark-brown solid, which could be decomposed at a temperature higher than 307°C (DTA/TG), was soluble in water, and had $\lambda_{max}$ of 392 nm (in an aqueous solution).

Example 66: 10.0 g of potassium hydroxyhematin was weighed and dissolved in 200 mL of water under stirring to produce a first solution. 11.0 g of manganese sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 8.12 g of **manganese hydroxyhematin.** The magnesium hydroxyhematin was a dark-brown solid, which could be decomposed at a temperature higher than 322°C (DTA/TG), was slightly soluble in water, and had $\lambda_{wax}$ of 390 nm (in an aqueous solution).

Example 67: 10.0 g of potassium hydroxyhematin was weighed and dissolved in 200 mL of water under stirring to produce a first solution. 14.0 g of ferrous sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 7.71 g of **ferrous hydroxyhematin.** The ferrous hydroxyhematin was a dark-brown solid, which could be decomposed at a temperature higher than 331°C (DTA/TG), was slightly soluble in water, and had $\lambda_{wax}$ of 371 nm (in an aqueous solution).

Example 68: 10.0 g of potassium hydroxyhematin was weighed and dissolved in 200 mL of water under stirring to produce a first solution. 11.0 g of ferric chloride was weighed and dissolved in 120 mL of water under stirring to produce a second solution. The first solution and the second solution were slowly mixed under stirring, then further stirred for 1 h, placed overnight, filtered, washed with cold water, and dried under reduced pressure to produce about 8.43 g of **ferric hydroxyhematin.** The ferric hydroxyhematin was a dark-brown solid, which could be decomposed at a temperature higher than 328°C (DTA/TG), was slightly soluble in water, and had $\lambda_{max}$ of 367 nm (in an aqueous solution).

Example 69: 10.0 g of potassium hydroxyhematin was weighed and dissolved in 200 mL of water under stirring to produce a first solution. 14.0 g of zinc sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 8.94 g of **zinc hydroxyhematin.** The zinc hydroxyhematin was a dark-brown solid, which could be decomposed at a temperature higher than 303°C (DTA/TG), was slightly soluble in water, and had $\lambda_{max}$ of 375 nm (in an aqueous solution).

Example 70: 10.0 g of potassium hydroxyhematin was weighed and dissolved in 200 mL of water under stirring to produce a first solution. 15.0 g of copper sulfate was weighed and dissolved in 150 mL of water under stirring to produce a second solution. The first solution and the second solution were slowly mixed under stirring, then further stirred for 1 h, placed overnight, filtered, washed with cold water, and dried under reduced pressure to produce about 8.22 g of **copper hydroxyhematin.** The copper hydroxyhematin was a dark-brown solid, which could be decomposed at a temperature higher than 266°C (DTA/TG), was slightly soluble in water, and had $\lambda_{max}$ of 363 nm (in an aqueous solution).

Example 71: 100.0 g of technical-grade iron chlorin (produced by Nanjing Baite Bioengineering Co., Ltd.) was weighed and dissolved in 1,000 mL of a solution of 6% potassium hydroxide in water under stirring, and stirring was further conducted in the dark for 5 h. 8,000 mL of acetone was slowly added, and stirring was conducted in the dark for 1 h to produce a mixed solution. The mixed solution was placed overnight, filtered, washed with cold acetone, and dried under reduced pressure to produce 9.02 g of **potassium iron hydroxychlorophyllin.** The potassium iron hydroxychlorophyllin was a dark-green powder, which could be decomposed at a temperature higher than 320°C (DTA/TG), was easily soluble in water, and had $\lambda_{max}$ of 395 nm (in an aqueous solution).

Example 72: This example was the same as Example 71, except that the potassium hydroxide aqueous solution was replaced with a sodium hydroxide aqueous solution, 8.46 g of **sodium iron hydroxychlorophyllin** was prepared, and the sodium iron hydroxychlorophyllin was a dark-green powder, which could be decomposed at a temperature higher than 311°C (DTA/TG), was easily soluble in water, and had $\lambda_{max}$ of 396 nm (in an aqueous solution).

Example 73: 5.0 g of sodium iron hydroxychlorophyllin was weighed and dissolved in 1,000 mL of water, and 5% sulfuric acid was slowly added dropwise under stirring in the dark until a pH was about 4 to produce a first mixed solution. The first mixed solution was placed overnight and filtered to produce a filter cake. The filter cake was washed with water until it was neutral and dried under reduced pressure to produce a solid. The solid was dissolved in 1,000 mL of acetone, and a dry ammonia gas was slowly introduced under stirring until it was saturated to produce a second mixed solution. The second mixed solution was placed overnight, filtered, washed with a small amount of cold acetone, and dried under reduced pressure to produce 3.34 g of **ammonium iron hydroxychlorophyllin.** The ammonium iron hydroxychlorophyllin was a dark-green powder, which could be decomposed at a temperature higher than 255°C (DTA/TG), was easily soluble in water, and had $\lambda_{max}$ of 404 nm (in an aqueous solution).

Example 74: 10.0 g of sodium iron hydroxychlorophyllin was weighed and dissolved in 100 mL of water under stirring to produce a first solution. 11.0 g of magnesium chloride hexahydrate was weighed and dissolved in 50 mL of water under stirring to produce a second solution. The first solution and the second solution were slowly mixed under stirring at room temperature to produce a precipitate. The precipitate was placed in the dark overnight, separated through filtration, washed 2 times with cold water, separated through pressure filtration, and then dried under reduced pressure to produce 7.27 g of **magnesium iron hydroxychlorophyllin.** The magnesium iron hydroxychlorophyllin was a dark-green powder, which could be decomposed at a temperature higher than 317°C (DTA/TG), was soluble in water, and had $\lambda_{max}$ of 401 nm (in an aqueous solution).

Example 75: 10.0 g of sodium iron hydroxychlorophyllin was weighed and dissolved in 100 mL of water under stirring to produce a first solution. 10.0 g of calcium chloride dihydrate was weighed and dissolved in 50 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 7.71 g of **calcium iron hydroxychlorophyllin.** The calcium iron hydroxychlorophyllin was a dark-green powder, which could be decomposed at a temperature higher than 332°C (DTA/TG), was soluble in water, and had $\lambda_{max}$ of 402 nm (in an aqueous solution).

Example 76: 10.0 g of sodium iron hydroxychlorophyllin was weighed and dissolved in 110 mL of water under stirring to produce a first solution. 10.0 g of ferrous chloride tetrahydrate was weighed and dissolved in 50 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 9.26 g of **ferrous iron hydroxychlorophyllin.** The ferrous iron hydroxychlorophyllin was a dark-green powder, which could be decomposed at a temperature higher than 315°C (DTA/TG), was slightly soluble in water, and had $\lambda_{max}$ of 398 nm (in an aqueous solution).

Example 77: 10.0 g of sodium iron hydroxychlorophyllin was weighed and dissolved in 110 mL of water under stirring to produce a first solution. 10.0 g of ferric chloride hexahydrate was weighed and dissolved in 50 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 8.11 g of **ferric iron hydroxychlorophyllin.** The iron iron hydroxychlorophyllin was a dark-green powder, which could be decomposed at a temperature higher than 304°C (DTA/TG), was slightly soluble in water, and had $\lambda_{max}$ of 401 nm (in an aqueous solution).

Example 78: 10.0 g of sodium iron hydroxychlorophyllin was weighed and dissolved in 100 mL of water under stirring to produce a first solution. 10.0 g of manganese chloride tetrahydrate was weighed and dissolved in 50 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 87.8 g of **manganese iron hydroxychlorophyllin.** The manganese iron hydroxychlorophyllin was a dark-green powder, which could be decomposed at a temperature higher than 331°C (DTA/TG), was slightly soluble in water, and had $\lambda_{wax}$ of 403 nm (in an aqueous solution).

Example 79: 10.0 g of sodium iron hydroxychlorophyllin was weighed and dissolved in 100 mL of water under stirring to produce a first solution. 10.0 g of zinc chloride was weighed and dissolved in 50 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 8.67 g of **zinc iron hydroxychlorophyllin.** The zinc iron hydroxychlorophyllin was a dark-green powder, which could be decomposed at a temperature higher than 327°C (DTA/TG), was slightly soluble in water, and had $\lambda_{wax}$ of 403 nm (in an aqueous solution).

Example 80: 10.0 g of sodium iron hydroxychlorophyllin was weighed and dissolved in 100 mL of water under stirring to produce a first solution. 9.0 g of copper chloride dihydrate was weighed and dissolved in 50 mL of water under stirring to produce a second solution. The first solution and the second solution were slowly mixed under stirring at room temperature to produce a precipitate. The precipitate was placed in the dark overnight, separated through filtration, washed with cold water, separated through pressure filtration, and then dried under reduced pressure to produce 8.42 g of **copper iron hydroxychlorophyllin.** The copper iron hydroxychlorophyllin was a dark-green powder, which could be decomposed at a temperature higher than 264°C (DTA/TG), was slightly soluble in water, and had $\lambda_{wax}$ of 395 nm (in an aqueous solution).

Example 81: DTA/TG for sodium iron chlorin and iron chlorin

Test instrument: Shimadzu DTG-60 DTA-TG synchronous analyzer.

Test samples: Sodium iron chlorin and iron chlorin.

Reference: Aluminum oxide.

Test gas flow: Nitrogen.

Heating rate: 10°C/min.

Heating range: 25°C to 500°C.

Sample weight: 4.3 mg to 4.5 mg.

Analysis software: ta60WS Ver. 2.11.

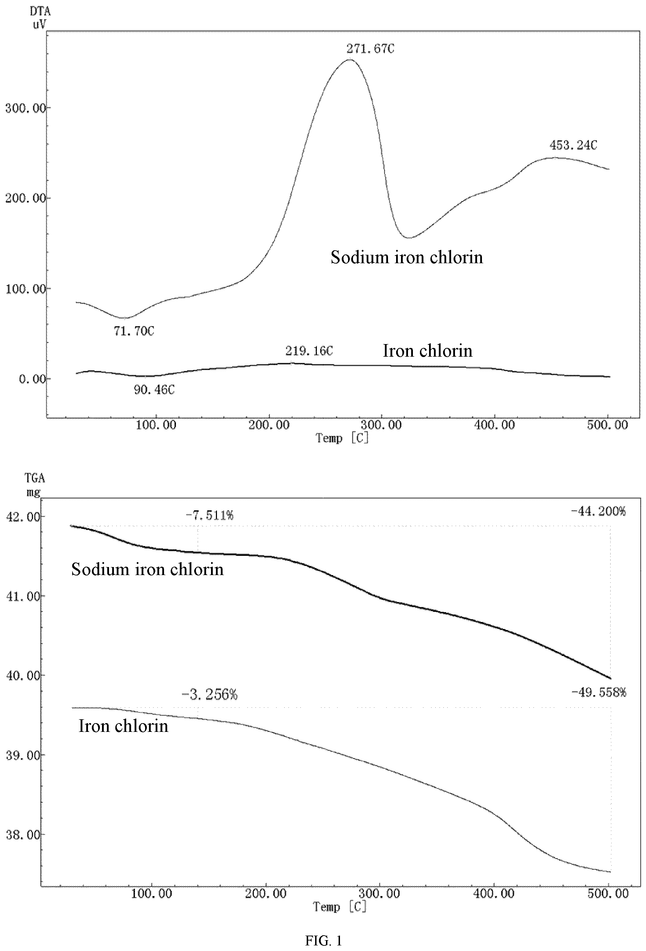

Result analysis: DTA/TG patterns of sodium iron chlorin and iron chlorin were determined, and patterns for the two samples were merged by the analysis software. Results were shown in FIG. 1, including a DTA comparison chart (the upper part in FIG. 1) and a TG comparison chart (the lower part in FIG. 1) for the samples.

It can be seen from this figure that DTA/TG patterns of the sodium iron chlorin and iron chlorin samples both had no melting phase-transition peak (no melting point), but indicated much different thermal responses. In a same measuring range (uV), a DTA curve of acidic iron chlorin (the upper part in FIG. 1) was smooth and did not fluctuate much and had an almost smooth endothermic peak/exothermic peak, while a DTA curve of sodium iron chlorin fluctuated greatly and had two large exothermic peaks at 271.67°C and 453.24°C. In the DTA curve of acidic iron chlorin, a peak at 90.46°C was an endothermic peak for the dehydration or the removal of low-boiling-point small molecule from the iron chlorin sample, a peak at 219.16°C was an exothermic peak for the decomposition of the iron chlorin sample, and a specific heat change of the iron chlorin sample in the measuring range was not obvious (approximate to a horizontal line). The dehydration of sodium iron chlorin involved significant heat absorption, an endothermic peak was shifted to 71.7°C, and a heat absorption capacity increased significantly. After 110°C, the specific heat of the sodium iron chlorin sample increased, and the DTA curve of the sodium iron chlorin sample arose. The decomposition of the sodium iron chlorin sample began at 180°C to cause the intense heat release. A first exothermic peak appeared at 271.67°C, and a second exothermic peak appeared at 453.24°C. After the further decomposition, the specific heat began to decrease. There was a very significant difference between thermal reactions of the above two samples, and the salt sample had a significantly-higher decomposition point than the acidic sample. According to the comparison of TG patterns (the lower part in FIG. 1): Before 140°C, a TG curve of iron chlorin was smooth and the iron chlorin sample had a small weight loss of 3.256%, while a TG curve of sodium iron chlorin descended rapidly and the sodium iron chlorin sample had a weight loss of 7.511% for dehydration, which was equivalent to a content of a dihydrate (salt). A weight loss (decomposition) of iron chlorin was slow after 180°C, and the weight loss was accelerated at 390°C (the decomposition was intensified). The sodium iron chlorin sample underwent a significant weight loss (decomposition) after 200°C, and a weight loss rate of the sodium iron chlorin sample began to decrease at 295°C and then began to increase (the decomposition was intensified) after 405°C. It indicated that the salt sample had a significantly-higher decomposition temperature than the acidic sample.

These results showed that the above two samples only had a decomposition point but no melting point under heat, and the sodium iron chlorin sample had significantly-stronger thermal stability than the acidic sample.

Example 82: Dilution and pH determination for sample solutions of sodium iron chlorin, etc.

Samples: Sodium iron chlorophyllin (commercially-available food additive, food grade, and content: 96%), protoporphyrin disodium (commercially-available pharmaceutical raw material, pharmaceutical grade, and content: 95.0%), sodium iron chlorin (content determined by spectrophotometry: 95.5%, the same below), potassium hematin (content: 96.0%), magnesium iron chlorophyllin (content: 97.3%), magnesium hematin (content: 95.7%), and purified water (pH = 7).

Instruments: An FA1604N electronic balance, a PHS-3C desktop acidity meter, an ultrasonic instrument, and volumetric flasks.

Method: 0.2500 g of a sample was accurately weighed and added to a 25 mL volumetric flask, dissolved with purified water, and diluted to a specified scale to prepare a 1.00% (10,000.0 ppm) initial solution. The initial solution was diluted with purified water in different ratios to prepare a series of solutions with a concentration of 1,000.0 ppm to 0.1 ppm. A pH value of each sample was determined three times. A slightly soluble salt was prepared into solutions according to the saturated solubility and the following concentrations: An appropriate amount of a sample was weighed, dissolved, and diluted and determined by the same method. mol/L concentrations corresponding to ppm concentrations and pH determination results for solutions were shown in the following table:

mol/L concentrations corresponding to different ppm concentrations and pH values for sample solutions

| Concentration (ppm) | mol/L concentration (pH) | | |
|---|---|---|---|
| | Sodium iron chlorin | Sodium iron chlorophyllin | Protoporphyrin disodium |
| 10000.0 | $1.36 \times 10^{-2}$ (10.41) | $1.44 \times 10^{-2}$ (10.87) | $1.65 \times 10^{-2}$ (10.60) |
| 1000.0 | $1.36 \times 10^{-3}$ (9.71) | $1.44 \times 10^{-3}$ (10.05) | $1.65 \times 10^{-3}$ (9.25) |
| 100.0 | $1.36 \times 10^{-4}$ (8.34) | $1.44 \times 10^{-4}$ (8.18) | $1.65 \times 10^{-4}$ (8.05) |
| 30.0 | $4.08 \times 10^{-5}$ (7.51) | $4.32 \times 10^{-5}$ (7.63) | $4.95 \times 10^{-5}$ (7.50) |
| 20.0 | $2.71 \times 10^{-5}$ (7.42) | $2.88 \times 10^{-5}$ (7.52) | $3.30 \times 10^{-5}$ (7.37) |
| 10.0 | $1.36 \times 10^{-5}$ (7.23) | $1.44 \times 10^{-5}$ (7.14) | $1.65 \times 10^{-5}$ (7.21) |
| 1.0 | $1.36 \times 10^{-6}$ (6.89) | $1.44 \times 10^{-6}$ (6.95) | $1.65 \times 10^{-6}$ (6.67) |
| 0.1 | $1.36 \times 10^{-7}$ (6.79) | $1.44 \times 10^{-7}$ (6.68) | $1.65 \times 10^{-7}$ (6.61) |
| | Potassium hematin | Magnesium hematin | Magnesium iron chlorophyllin |
| 10000.0 | $1.37 \times 10^{-2}$ (10.21) | -- | -- |
| 1000.0 | $1.37 \times 10^{-3}$ (9.43) | -- | -- |
| 100.0 | $1.37 \times 10^{-4}$ (8.10) | $1.48 \times 10^{-4}$ (8.01) | $1.49 \times 10^{-4}$ (8.18) |
| 30.0 | $4.11 \times 10^{-5}$ (7.57) | $4.44 \times 10^{-5}$ (7.87) | $4.47 \times 10^{-5}$ (7.78) |
| 20.0 | $2.74 \times 10^{-5}$ (7.45) | $2.96 \times 10^{-5}$ (7.77) | $2.98 \times 10^{-5}$ (7.65) |
| 10.0 | $1.37 \times 10^{-5}$ (7.33) | $1.48 \times 10^{-5}$ (7.63) | $1.49 \times 10^{-5}$ (7.40) |
| 1.0 | $1.37 \times 10^{-6}$ (6.90) | $1.48 \times 10^{-6}$ (7.27) | $1.49 \times 10^{-6}$ (7.11) |
| 0.1 | $1.37 \times 10^{-7}$ (6.85) | $1.48 \times 10^{-7}$ (6.92) | $1.49 \times 10^{-7}$ (6.94) |

As shown in the table: Among the tested samples, a solution of an easily soluble sodium/potassium salt at a concentration of 1% (10,000.0 ppm) has a pH of 10.21 to 10.95, indicating strong alkalinity, and the solution is still strongly-alkaline after being 10 fold-diluted. With the further decrease of the concentration, the pH decreases significantly. A solution of a soluble magnesium salt (100 ppm) has a similar pH to the solution of the sodium/potassium salt. A solution of a sample at 30.0 ppm has a pH of less than 8.0 (pH = 7.50 to 7.63), which is in a pH range for the physiological adaptation of plants. When diluted to 10.0 ppm, a sample solution is nearly neutral. When further diluted to the concentration range of 1.0 ppm to 0.1 ppm (about $10^{-6}$ mol/L) specified in the present disclosure, a sample solution is unexpectedly weakly-acidic. Among the tested samples, sodium iron chlorophyllin (food additive) and protoporphyrin disodium (drug for treating liver diseases) are commercially-available products. Like the samples such as sodium iron chlorin and sodium hematin newly prepared by the present disclosure, dilute solutions of sodium iron chlorophyllin and protoporphyrin disodium all are unexpectedly weakly-acidic (not reported in literature).

Generally, when an acidic or alkaline solution is diluted until a hydrolytically-dissociated hydrogen ion concentration is close to $10^{-7}$ mol/L or lower, a pH of a solution will be close to or approximately equal to 7.0, but will not exceed 7.0 (turning from acidic to alkaline or from alkaline to acidic). Due to the hydrolysis of weak acid groups, an aqueous solution of a strong alkali-weak acid salt at a general concentration (concentration: much higher than $10^{-7}$ mol/L) is obviously alkaline. When the solution is diluted, with the decrease of the concentration, a hydrolysis effect for the salt is gradually weakened. When the solution is diluted to $10^{-6}$ mol/L to $10^{-7}$ mol/L (a sample concentration in this example is 1.0 ppm to 0.1 ppm), hydrogen ions resulting from hydrolytic dissociation are gradually dominant. In this case, a pH of the solution should be close to or approximately equal to 7.0, but a pH of the solution determined actually is lower than 7.0, indicating that the solution unexpectedly turns from weakly-alkaline to weakly-acidic. This result indicates that there are acidic components in the solution. That is, the tested porphin salt sample may include a small amount/trace amount of coexisting acidic porphin ingredients, and these residual acidic porphin ingredients are important factors for the weak acidity of a diluted solution.

Acidic porphin is a polybasic acid. For example, protoporphyrin disodium and heme are dibasic acids, and chlorin and chelates thereof each are a mixture of monobasic acid, dibasic acid, and tribasic acid monomers. In the present disclosure, the soluble sodium, potassium, or ammonium salt samples are prepared by subjecting acidic porphin and an alkali to a neutralization reaction in an organic solvent for precipitation, and divalent and trivalent metal salts such as magnesium salts are prepared through double decomposition reaction-based precipitation. In a salt-producing reaction process, a variety of process factors such as a component concentration, an alkali addition speed, stirring uniformity, and a reaction temperature restrict the generation of a precipitate and the composition of a product. Experiments have proved that the salt generation in a preparation process of a porphin salt is incomplete, or a small number of unreacted acidic molecules are included in a precipitate, resulting in the phenomenon of co-precipitation. In addition, the characteristic that planar porphin molecules easily aggregate with each other is the internal reason for the easy co-precipitation. Therefore, the salt generation is inevitably incomplete during the preparation. Such an unexpected pH change improves the practicability of the product of the present disclosure, is suitable for the physiological conditions of crops, and facilitates the application in field crops.

Example 83: Determination of stability of sample solutions in the dark/under light

Samples: 90 porphin salt and chlorin salt samples (contents: all higher than or equal to 95.0%, which were determined by spectrophotometry).

Reference substances: Iron chlorin and chlorhematin.

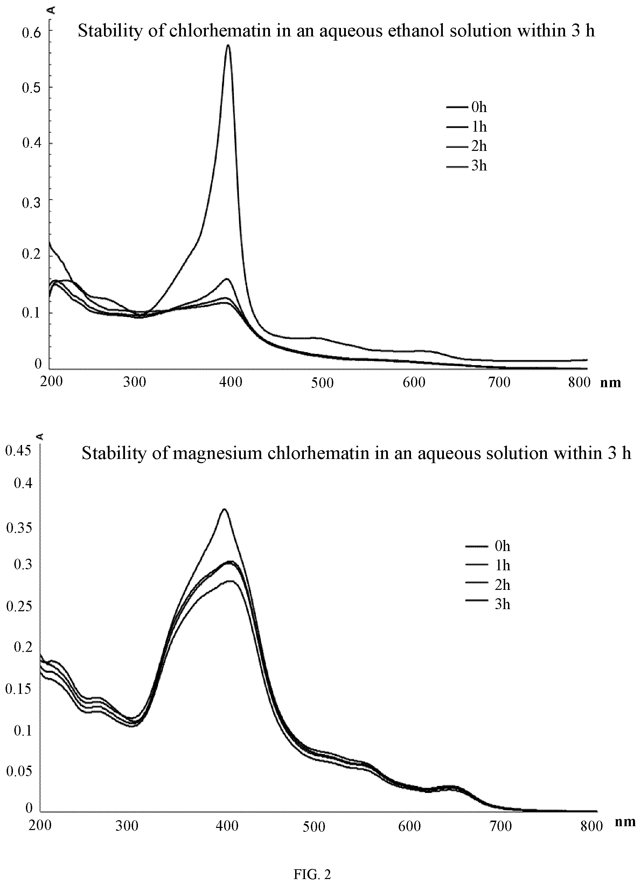

Instruments: A Shimadzu UV2600 ultraviolet spectrophotometer and a SMART AR813A illuminance meter.
Determination: An appropriate amount of a salt sample was taken, dissolved with water, and diluted to prepare a test solution with a concentration of about 10 ppm. An acidic reference substance (extremely-insoluble in water) was prepared with a 30% to 50% ethanol aqueous solution into a test solution with a concentration of about 10 ppm.
Each test solution was scanned by an ultraviolet spectrophotometer (at 0 h) for absorbance in a range of 200 nm to 800 nm immediately after being prepared. Then a test solution of each sample was divided into two parts. One part was placed at room temperature of 25°C in the dark, and the other part was placed under light with a light intensity of 30,000 lux. At 1 h, 2 h, and 3 h, a solution sample was collected and scanned for absorbance respectively.
An absorbance value of a maximum absorption peak at 0 h (starting) of each test solution was set as a relative content of 100%, and a ratio of an absorbance value at each time point to the absorbance value at 0 h was calculated to determine a relative content, %.
Results: In the dark, contents of the salt samples and the acidic reference substance samples to be tested all remained basically unchanged or slightly decreased (but a relative content was still higher than 96.5%) individually.
In the comparative test under light, there was a very significant difference in stability between a salt sample solution and a reference substance sample. Absorbance scanning results for stability of merely chlorhematin and magnesium chlorhematin solutions as examples under light were shown in FIG. 2: a relative content of the chlorhematin solution was reduced to less than 30% after being exposed to light for 1 h, and was only 20.21% after being exposed to light for 3 h. Correspondingly, a relative content of the magnesium chlorhematin solution was 75.61% after being exposed to light for 3 h, which was much higher than the relative content of the acidic reference substance. The comparison between iron chlorin and a sodium salt thereof also had similar results. Relative content changes of compared samples were shown in the following table:

Relative contents (%) of sample solutions in the dark/under light for 3 h

| Sample | Time (h) | In the dark | | Under light | |
|---|---|---|---|---|---|
| | | Acidic | Salt | Acidic | Salt |
| Chlorhematin (magnesium salt) | 0 | 100.00 | 100.00 | 100.00 | 100.00 |
| | 1 | 99.55 | 100.10 | 27.73 | 82.11 |
| | 2 | 99.24 | 99.76 | 21.64 | 81.84 |
| | 3 | 98.65 | 99.38 | 20.21 | 75.61 |
| Iron chlorin (sodium salt) | 0 | 100.0 | 100.0 | 100.00 | 100.00 |
| | 1 | 99.25 | 99.51 | 31.04 | 69.35 |
| | 2 | 99.26 | 99.60 | 28.12 | 59.05 |
| | 3 | 99.20 | 99.43 | 23.73 | 54.02 |

Further, a time required for the reduction of a relative content by 50% (half-life $t_{1/2}$) for each sample solution under strong light was further measured and calculated, and results were shown in the following table:

Half-life $t_{1/2}$ for a relative content of each sample solution in the dark/under light (h)

| Sample | In the dark | | Under light | |
|---|---|---|---|---|
| | Acidic | Salt | Acidic | Salt |
| Chlorhematin (magnesium salt) | 114.70 | 257.54 | 0.69 | 6.00 |
| Iron chlorin (sodium salt) | 176.92 | 277.17 | 0.73 | 3.41 |

Results: Half-life ($t_{1/2}$) values for contents of the reference substances chlorhematin and iron chlorin were 0.69 h and 0.73 h, respectively, and half-life values for magnesium and sodium salts of chlorhematin and iron chlorin increased to 6.0 h and 3.41 h, respectively, with increases of 8.69 times and 4.67 times, respectively. The light stability of a salt compound solution was significantly enhanced compared with an acidic control. In the dark, a salt compound solution also had significantly-longer $t_{1/2}$ than an acidic compound solution, indicating that a salt compound exhibited high stability, was suitable for the complex application environment of the field, and had a high practical use value.

The stability of aqueous solutions of 90 salt compounds (measured concentration: 12 ppm to 25 ppm) under 3 h strong light, and results were shown in the following table:

Changes in relative contents (%) of a series of salt sample solutions under 1 h to 3 h light

| Sample | Time h | Relative content/%* | | | | | | | | | |
|---|---|---|---|---|---|---|---|---|---|---|---|
| | | $Na^+$ | $K^+$ | $NH_4^+$ | $Mg^{2+}$ | $Ca^{2+}$ | $Fe^{2+}$ | $Fe^{3+}$ | $Mn^{2+}$ | $Zn^{2+}$ | $Cu^{2+}$ |
| Protoporphyrin salt | 1 | 63.47 | 65.90 | 60.29 | 93.32 | 90.07 | 85.02 | 91.31 | 92.57 | 90.95 | 96.42 |
| | 2 | 62.74 | 56.97 | 48.76 | 90.98 | 87.11 | 79.78 | 80.04 | 90.09 | 89.52 | 95.56 |
| | 3 | 57.05 | 58.17 | 45.99 | 89.65 | 85.89 | 78.28 | 79.25 | 88.85 | 86.51 | 94.88 |
| Chlorhematin | 1 | 74.11 | 90.91 | 73.07 | 82.11 | 82.53 | 96.12 | 96.13 | 83.39 | 93.45 | 95.23 |
| | 2 | 69.68 | 90.08 | 67.80 | 81.84 | 81.78 | 94.83 | 90.98 | 82.32 | 93.10 | 95.03 |
| | 3 | 67.73 | 81.82 | 66.25 | 75.61 | 81.97 | 85.46 | 92.45 | 81.07 | 92.74 | 93.04 |
| Iron chlorin | 1 | 69.35 | 70.13 | 63.54 | 74.25 | 76.78 | 65.57 | 71.06 | 72.13 | 71.19 | 78.52 |
| | 2 | 59.05 | 62.66 | 61.41 | 66.34 | 67.79 | 61.68 | 67.32 | 68.26 | 65.23 | 71.02 |
| | 3 | 54.02 | 59.17 | 55.37 | 62.11 | 60.67 | 57.23 | 63.29 | 63.87 | 61.37 | 64.29 |
| Iron chlorophyllin | 1 | 61.61 | 72.04 | 62.53 | 71.60 | 81.00 | 64.25 | 63.12 | 70.13 | 69.34 | 72.13 |
| | 2 | 54.63 | 65.63 | 58.11 | 62.44 | 74.21 | 61.00 | 61.14 | 61.00 | 63.04 | 68.58 |
| | 3 | 53.29 | 57.54 | 51.07 | 63.14 | 63.35 | 57.00 | 56.68 | 58.68 | 60.46 | 65.03 |
| Chlorin salt | 1 | 41.71 | 60.05 | 52.19 | 43.47 | 51.17 | 42.65 | 55.46 | 44.94 | 55.66 | 85.27 |
| | 2 | 30.78 | 46.81 | 47.37 | 35.26 | 48.34 | 38.87 | 43.21 | 40.28 | 50.60 | 82.15 |
| | 3 | 26.96 | 42.89 | 41.33 | 30.41 | 42.83 | 30.67 | 40.77 | 34.21 | 45.30 | 80.17 |
| Zinc chlorophyllin | 1 | 51.90 | 52.48 | 56.43 | 53.53 | 60.41 | 87.50 | 79.19 | 58.33 | 71.62 | 88.43 |
| | 2 | 42.51 | 46.08 | 50.21 | 45.74 | 48.22 | 54.08 | 74.35 | 50.00 | 66.31 | 93.28 |
| | 3 | 37.81 | 41.51 | 48.37 | 39.42 | 42.39 | 43.11 | 68.72 | 44.70 | 63.93 | 70.90 |
| Copper chlorophyllin | 1 | 72.50 | 83.53 | 87.29 | 74.46 | 75.63 | 77.19 | 76.52 | 76.34 | 69.84 | 98.85 |
| | 2 | 67.03 | 77.11 | 83.44 | 68.03 | 70.42 | 42.11 | 71.03 | 72.11 | 65.37 | 97.47 |
| | 3 | 66.30 | 78.11 | 79.81 | 66.86 | 64.38 | 22.37 | 67.21 | 67.37 | 60.04 | 96.78 |
| Hydroxyhematin salt | 1 | 70.21 | 77.06 | 75.37 | 96.74 | 86.39 | 95.45 | 96.01 | 87.45 | 94.32 | 96.20 |
| | 2 | 62.33 | 75.72 | 64.41 | 93.78 | 85.55 | 93.03 | 92.44 | 83.77 | 93.24 | 95.71 |
| | 3 | 57.03 | 73.04 | 61.27 | 93.33 | 75.80 | 87.63 | 91.07 | 80.00 | 90.85 | 92.81 |
| Iron hydroxychlorophyll in | 1 | 60.39 | 62.96 | 64.04 | 80.48 | 81.89 | 72.56 | 73.34 | 75.51 | 73.29 | 79.89 |
| | 2 | 52.14 | 54.32 | 60.10 | 67.53 | 74.32 | 70.04 | 69.87 | 70.44 | 69.57 | 76.11 |
| | 3 | 47.32 | 49.38 | 52.18 | 67.13 | 62.43 | 66.80 | 64.93 | 68.12 | 65.55 | 73.21 |

*: a relative content of 100% at 0 h.

Results: The series of salt compounds of the present disclosure all exhibited strong stability. Relative contents of most of the sample solutions after being exposed to light for 3 h could still be maintained at 50% or more. The salt compounds of the present disclosure exhibited significantly-better stability than the existing acidic products. Therefore, the salt compounds of the present disclosure can well meet the actual extensive needs and have high practical values.

Example 84: Impacts of sodium iron chlorin, potassium hematin, and potassium protoporphyrin on the germination of wheat and the growth of seedlings

Test variety: Huaimai 35

Samples: Sodium iron chlorin, potassium hematin, potassium protoporphyrin, and clear water control (CK).

Method: 3 replicates were set for each treatment. 50 full seeds with a consistent size were selected each time for each sample, disinfected with sodium hypochlorite for 5 min, rinsed with clear water, and placed in a plastic box filled with 100 g of a sand. 25 mL of a prepared sample solution was poured into the plastic box, and the seeds were arranged regularly. The plastic box was covered with a lid, and then incubated in a 25°C incubator. The germination was determined when a length of a sprout of a wheat seed exceeded a half of a diameter (long axis) of the wheat seed (determination criterion). According to the growth of wheat, the germ exposure of seeds and the number of germinated seeds were recorded every day. On day 6, 10 wheat seedlings were randomly selected, and a root length, a plant height, and a root number were determined. On day 7, a root weight and an aboveground part weight were determined. All index data was produced based on 3 replicates.

Germination rates of wheat in the three samples at different concentrations were shown in Table 1 below:

Table 1 Germination rate of wheat at each sample concentration

| Sample concentration/ppm | Sodium iron chlorin (%) | Potassium hematin (%) | Potassium protoporphyrin (%) |
|---|---|---|---|
| 0 | 92.7 | -- | -- |
| 0.2500 | 93.3 | 97.3 | 90.7 |
| 0.0250 | 95.3 | 94.7 | 95.3 |
| 0.0025 | 94.0 | 94.0 | 94.7 |

Results: Compared with the control (CK = 0 ppm), each sample in a concentration range of 0.2500 ppm to 0.0025 ppm exhibited a significant effect of promoting the wheat germination, where potassium hematin at 0.2500 ppm exhibited the optimal effect of promoting the germination rate, and other samples exhibited a prominent effect at a concentration of 0.0250 ppm.

Impacts of sodium iron chlorin solutions with different concentrations on wheat seedling roots/seedlings were shown in Table 2 below:

Table 2 Influence of sodium iron chlorin on the growth of wheat seedlings

| Sample concentration/ppm | Root number | Root length (cm) | Root weight (g) | Seedling height (cm) | Seedling weight (g) |
|---|---|---|---|---|---|
| 0 | 5.1 | 9.82 | 0.0973 | 9.30 | 0.179 |
| 0.2500 | 4.9 | 9.64 | 0.0867 | 7.95 | 0.145 |
| 0.0250 | 5.2 | 10.17 | 0.1027 | 9.13 | 0.178 |
| 0.0025 | 5.3 | 9.30 | 0.0953 | 8.88 | 0.170 |

Results: The different concentrations of sodium iron chlorin had different impacts on the growth of early wheat seedlings, and exhibited a significant effect of promoting the growth of a root system, but exhibited a relatively weak impact on the aboveground seedling part. 0.0250 ppm was determined as a suitable concentration.

Impacts of potassium hematin solutions with different concentrations on wheat seedling roots/seedlings were shown in Table 3 below:

Table 3 Impacts of potassium hematin at different concentrations on the growth of wheat seedlings

| Sample concentration/ppm | Root number | Root length (cm) | Root weight (g) | Seedling height (cm) | Seedling weight (g) |
|---|---|---|---|---|---|
| 0 | 5.1 | 11.02 | 0.1147 | 8.96 | 0.257 |
| 0.2500 | 5.3 | 9.15 | 0.1036 | 7.51 | 0.211 |
| 0.0250 | 5.2 | 9.74 | 0.1143 | 9.56 | 0.286 |
| 0.0025 | 5.1 | 10.01 | 0.1163 | 9.42 | 0.298 |

Results: Potassium hematin at a low concentration (0.0250 ppm or 0.0025 ppm) could significantly promote the growth of aboveground parts of wheat seedlings and the increase of a root number, but exhibited a relatively weak effect of promoting a root length.

Impacts of potassium protoporphyrin solutions with different concentrations on wheat seedling roots/seedlings were shown in Table 4 below:

Table 4 Impacts of potassium protoporphyrin at different concentrations on the growth of wheat seedlings

| Sample concentration/ppm | Root number | Root length (cm) | Root weight (g) | Seedling height (cm) | Seedling weight (g) |
|---|---|---|---|---|---|
| 0 | 5.3 | 9.32 | 0.1160 | 9.19 | 0.253 |
| 0.2500 | 5.3 | 8.68 | 0.1134 | 7.64 | 0.228 |
| 0.0250 | 5.2 | 10.17 | 0.0976 | 9.48 | 0.242 |
| 0.0025 | 5.4 | 9.63 | 0.1230 | 8.95 | 0.241 |

Results: Potassium protoporphyrin at a low concentration (0.0250 ppm or 0.0025 ppm) exhibited a specified effect of promoting the growth of wheat root systems, but exhibited a relatively weak effect of promoting the growth of aboveground parts of seedlings.

Example 85: *In vitro* chlorophyllase-inhibiting activity test According to the method in the literature (Acta Botanica Boreali-Occidentalia Sinica, 2003, 23 (5): 750-754.), 2.00 g of an acetone powder made of mung bean leaves was accurately weighed and subjected to extraction in 50 mL of phosphate buffered saline (pH: 7.0 to 7.3) for 4.0 h to produce an extraction solution, and then the extraction solution was centrifuged at 4°C to 5°C for 10 min to produce a supernatant, which was a chlorophyllase solution with an active enzyme concentration of 0.13 mmol/L. A chlorin salt sample was added at a concentration of 0.3 ppm to 30.0 ppm to the chlorophyllase solution, and then a substrate chlorophyll (120 ppm) was added. A chlorophyllase inhibition rate of a salt sample solution at each concentration was determined, and an $IC_{50}$ value for a chlorophyllase inhibition activity of a salt sample was calculated. Results were shown in the following table:

$IC_{50}$ for chlorophyllase inhibition activities of a series of salt samples

| Sample | $IC_{50}$ (ppm) | | | | | | | | | |
|---|---|---|---|---|---|---|---|---|---|---|
| | $Na^+$ | $K^+$ | $NH4^+$ | $Mg^{2+}$ | $Ca^{2+}$ | $Fe^{2+}$ | $Fe^{3+}$ | $Mn^{2+}$ | $Zn^{2+}$ | $Cu^{2+}$ |
| Iron chlorin | 5.22 | 5.07 | 5.58 | 5.06 | 5.11 | 5.01 | 5.17 | 5.20 | 5.66 | 4.92 |
| Iron chlorophyllin | 5.41 | 5.09 | 5.43 | 5.26 | 5.38 | 5.27 | 5.35 | 5.55 | 5.16 | 5.04 |
| Chlorin salt | 6.03 | 5.98 | 6.05 | 6.22 | 6.43 | 6.25 | 5.71 | 6.23 | 6.37 | 6.18 |
| Zinc chlorophyllin | 15.91 | 15.55 | 18.06 | 16.37 | 17.78 | 18.29 | 16.43 | 18.10 | 18.77 | 17.76 |
| Copper chlorophyllin | 13.72 | 12.89 | 13.68 | 11.87 | 13.49 | 13.22 | 14.34 | 13.85 | 14.19 | 13.70 |

The chlorin salt retained the strong chlorophyllase inhibition activity of acidic chlorin, which is of great significance and values for practical applications.

Example 86: Experiment of inducing adventitious roots from hypocotyls of mung bean

Test solution preparation: 8 mL of a commercially-available STANLEY universal hydroponic nutrient solution was taken and diluted with tap water to 3,200 mL (400-fold dilution) to produce a dilute nutrient solution. An appropriate amount of each of salt samples such as protoporphyrin disodium and potassium chlorhematin was weighed and prepared with the dilute nutrient solution into 0.010 ppm and 0.1 ppm sample test solutions, and 150 mL of each test solution was taken. 150 mL of the dilute nutrient solution was taken as a blank control. The test solutions and the control solution were placed in brown bottles, and the brown bottles were sealed with a black film. Mung bean seedlings with uniform growth and a seedling height of 7 cm to 8 cm were selected, and cut at 3 cm below a first true leaf node. 8 mung bean seedlings were inserted into each bottle (with roots removed) to make lower radicles soaked in a solution. Mung bean seedlings were treated at room temperature of 20°C to 25°C for 7 d in total, during which the solution was changed every 2 d. On day 2 to day 3 of the treatment, there was a significant difference. On day 7, the observation and determination were conducted. An average adventitious root number and an average total adventitious root length for each seedling were calculated, and results were shown in the following table:

| Experimental group | Concentration (ppm) | Adventitious root number (root/seedling) | Total adventitious root length (cm/seedling) |
|---|---|---|---|
| Water | 0 | 9.26 | 15.35 |

(continued)

| Experimental group | Concentration (ppm) | Adventitious root number (root/seedling) | Total adventitious root length (cm/seedling) |
|---|---|---|---|
| Protoporphyrin disodium | 0.1 | 9.77 | 21.30 |
| Potassium chlorhematin | 0.1 | 14.02 | 25.21 |
| Potassium iron chlorin | 0.1 | 14.54 | 23.55 |
| Sodium iron chlorophyllin | 0.1 | 13.67 | 19.40 |
| Chlorhematin | 0.1 | 10.33 | 18.28 |
| Protoporphyrin disodium | 0.01 | 9.61 | 20.74 |
| Potassium chlorhematin | 0.01 | 14.85 | 26.83 |
| Potassium iron chlorin | 0.01 | 15.05 | 24.56 |
| Sodium iron chlorophyllin | 0.01 | 14.51 | 20.06 |
| Chlorhematin | 0.01 | 11.07 | 18.03 |

Results: Compared with the blank control, each test sample had a significant effect of promoting a rooting range and a root number from hypocotyls of mung bean, and the effect was better than an effect of the existing plant growth regulator product: chlorhematin.

Example 87: Experiment of controlling sheath blight in rice to improve a yield (use as a plant immunity inducer)

Site: Zhonghua Family Farm, Guangming Village, Dazhong Street, Dafeng District, Jiangsu Province. 6 mu of a clay loam field with a flat terrain and a prominent fertility was selected. A fore-rotating crop was wheat.

Experimental rice variety: Nanjing 9108.

The sowing was conducted on May 16, 2021, and the transplanting was conducted on June 12, 2021. The mechanized transplanting was conducted with a plant spacing of 18 cm and a row spacing of 25 cm.

According to the field efficacy standard of GB/T17980.20-2000, the following two treatments were set: a first treatment: sodium iron chlorin was applied at 3.0 mg/mu, and a second treatment: clear water was applied (control). Based on the characteristics of plant growth and the needs of disease and pest control, an agent was sprayed once at each of a late tillering stage of (July 22, 2021), a jointing-booting stage (August 16, 2021), and a full heading stage (August 30, 2021), with water amounts of 30 L, 45 L, and 45 L (concentrations: 0.100 ppm, 0.067 ppm, and 0.067 ppm) per mu, respectively. A test sample was dissolved, diluted, thoroughly stirred, and then applied, with an application area of 3 mu for each treatment.

Before harvesting, five-point sampling (20 m$^2$ per sampling point) was conducted for each treatment to determine the details of sheath blight in rice. Resulting corrected control effects were shown in the following table:

| Treatment | Diseased plant rate/% | Disease index/% | Corrected control/% |
|---|---|---|---|
| Sodium iron chlorin | 10.4 | 21.61 | 45.16 |
| Control | 60.8 | 39.38 | -- |

The harvesting was conducted on November 8, 2021. On the eve of harvesting, the yield composition and structure were investigated. Then a yield per mu was measured. Results were as follows:

| Treatment | Ear number per mu (10,000) | Filled grain number per ear | Thousand-grain weight (g) | Yield (kg/mu) | Yield increase rate (%) |
|---|---|---|---|---|---|
| Sodium iron chlorin | 26.14 | 99.93 | 27.00 | 705.38 | 11.46 |
| Control | 23.89 | 98.17 | 26.99 | 632.83 | - |

It can be seen from this experiment that sodium iron chlorin had a significant effect of controlling sheath blight in rice, and also exhibited a prominent effect of increasing a yield of rice, indicating that sodium iron chlorin is a plant immunity inducer with excellent performance.

Example 88: Experiment of controlling leaf blast in rice to improve a yield (use as a plant immunity inducer)

Site: Zhonghua Family Farm, Guangming Village, Dazhong Street, Dafeng District, Jiangsu Province. A fore-rotating crop was oilseed rape.

Experimental plot: flat terrain, clay loam, and excellent fertility.

Rice variety: Nanjing 9108.

The sowing was conducted on May 16, 2021, and the transplanting was conducted on June 14, 2021. The mechanized transplanting was conducted with a plant spacing of 18 cm and a row spacing of 25 cm.

The experiment was conducted according to experimental specifications in the field efficacy standard of GB/T17980.19-2000. In the experiment, the following two treatments were set: a first treatment: sodium iron chlorin was applied, and a second treatment: a blank control (water) was applied. A test sample was fully dissolved and diluted, and then applied. Based on the characteristics of disease and pest control, an agent was applied once at each of a late tillering stage (July 22, 2021), a jointing-booting stage (August 16, 2021), and a full heading stage (August 30, 2021). Sodium iron chlorin was applied at 3 mg/mu. Water amounts required per mu for the three times were 30 L, 45 L, and 45 L (sample concentrations: 0.1 ppm, 0.067 ppm, and 0.067 ppm), respectively. An area for each treatment was 3 mu. According to a five-point sampling method, 5 sampling points (20 m$^2$ per sampling point) were set for each treatment. The occurrence of leaf blast in rice was investigated in the field before harvesting. Corrected control effects were shown in the following table:

| Treatment | Average incidence rate (%) | Average disease index (%) | Corrected control effect (%) |
|---|---|---|---|
| Sodium iron chlorin | 8.0 | 20.09 | 43.93 |
| Control | 38.4 | 35.82 | - |

The experimental results showed that sodium iron chlorin could significantly improve the resistance of rice to leaf blast, and thus could be used as a plant immunity inducer.

The harvesting was conducted on November 8, 2021. On the eve of harvesting, the yield composition and structure were investigated. Then a yield per mu was measured. The sample exhibited a significant effect of increasing a yield. Specific results were shown in the following table:

| Treatment | Ear number per mu (10,000) | Filled grain number per ear | Thousand-grain weight (g) | Yield (kg/mu) | Yield increase rate (%) |
|---|---|---|---|---|---|
| Sodium iron chlorin | 22.40 | 117.39 | 26.9 | 616.85 | 7.1 |
| Control | 20.40 | 111.79 | 26.65 | 575.92 | -- |

Example 89: Experiment of spraying a potassium hematin solution on pepper to increase a yield

Experimental site: Litangfang Village, Wucheng Town, Wucheng County, Dezhou City.

Experimental plot: medium loam, and medium to high fertility. A fore-rotating crop was sorghum.

Test variety: Hebei Jize pepper. Planting density: 3,500 trees/mu.

Planting mode: mechanized transplanting.

Planting time: May 8, 2020.

Experimental method: A land area of 10 mu was adopted for each of experimental and control groups. A treatment group was sprayed with a potassium hematin solution, a control group was sprayed with heme (at the same dose concentration as the treatment group), and a blank was sprayed with clear water. On May 19 (after transplanting) and July 2 (blooming stage), an agent was sprayed at 1.5 mg/mu in the treatment and control groups, with water amounts per mu respectively as follows: 15 L and 30 L (sample concentrations: 0.1 ppm and 0.05 ppm). On September 13, 10 pepper plants with uniform growth were selected from each of the treatment group and the control group, and fruit numbers and single-fruit weights were counted. The whole field was harvested in three batches on August 30, September 13, and September 25, respectively. Yields were subjected to combined statistics and comparison, and results were as follows:

| Treatment | Average fruit number (fruits/plant) | Single-fruit weight (g/fruit) | Yield per mu (kg/mu) | Yield increase rate (%) |
|---|---|---|---|---|
| Potassium hematin | 50.5 | 18.5 | 2975.0 | 10.59 |
| Heme | 49.2 | 18.6 | 2833.3 | 5.33 |
| Clear water | 47.8 | 17.9 | 2690.0 | -- |

The experiment showed that the spraying of potassium hematin on pepper could significantly increase a fruit setting rate and a single-fruit weight and exhibited a significant yield-increasing effect, and the control group heme also exhibited a prominent yield-increasing effect, but the yield-increasing effect of heme was significantly inferior to the yield-increasing effect of potassium hematin.

Example 90: Experiment of spraying sodium iron chlorophyllin on tomato to increase a yield

Experimental site: 3 connected plots of the 22nd Regiment of the Xinjiang Production and Construction Corps, which were nurseries previously and had a light loam soil texture.

Variety: Jinfan 1615.

During cultivation, flat planting, plastic mulching, seedling transplanting, and pressurized drip irrigation were adopted. On April 29, seedlings were transplanted at 2,600 seedlings/mu. An area of 20 mu was adopted for each of control and treatment groups. Each treatment region was sprayed with a sodium iron chlorophyllin solution at a concentration of 0.05 ppm twice in an early flowering stage (May 22, 2021) and a fruit setting stage (June 5, 2021), respectively.

The harvesting was conducted on August 3. During the harvesting, 5 plants were continuously and randomly selected from each treatment for sampling statistics. Data was as follows:

| Treatment | Ear number/plant | Red fruit number/plant (fruit) | Green fruit number/plant (fruit) | Average single-fruit weight (g) | Yield per mu (kg/mu) | Yield increase rate (%) |
|---|---|---|---|---|---|---|
| Sodium iron chlorophyllin | 6.9 | 46.4 | 13.8 | 51.3 | 6188.8 | 27.15 |
| Control | 4.8 | 40 | 12.6 | 46.8 | 4867.2 | -- |

Results showed that sodium iron chlorophyllin could increase a fruit setting rate and a single-fruit weight for tomato, thereby increasing a yield per mu for tomato by more than 27%. In addition, it was observed that a red-to-green fruit ratio of tomato sprayed with sodium iron chlorophyllin reached 3.36 (a red-to-green fruit ratio for the control was 3.17), and the red-to-green fruit ratio of tomato sprayed with sodium iron chlorophyllin increased by 5.91% compared with the control, indicating that sodium iron chlorophyllin could not only increase a yield, but also exhibit a significant effect of promoting the early coloring of tomato fruits to improve a quality of the fruits. The experiment proved that sodium iron chlorophyllin had a high practical value and a promising application prospect.

Example 91: Comparative experiment of spraying iron chlorin and a potassium salt thereof on wheat to increase a yield

Experimental site: Wheat experimental plots of the Agricultural Research Institute, Huanghai Farm, Jiangsu Province. A fore-rotating crop was:
rice, where sowing time: December 10, 2020, and sowing amount: 30 kg/mu.

Crop: Huaimai 35. An iron chlorin (a powder) treatment plot, a potassium salt treatment plot, and a control plot (sprayed with clear water) each were of 7 mu.

Method: An iron chlorin/potassium salt solution was sprayed once at each of a three-true leaf and spear leaf stage (March 14, 2021), an inflorescence emergence stage (April 14, 2021), and a flowering stage (May 1, 2021). The iron chlorin/potassium salt was sprayed at 3 mg/mu each time, with the following water amounts per mu: 15 L, 30 L, and 30 L (concentrations: 0.2 ppm, 0.1 ppm, and 0.1 ppm), respectively.

On June 11, 2021, the harvesting was conducted throughout the field at a time. Measured yield data was as follows:

| Treatment | Ear number per mu (10,000) | Grain number per ear (grains/car) | Thousand-grain weight (g) | Actual yield per mu (kg) | Yield increase rate per mu (%) |
|---|---|---|---|---|---|
| Potassium iron chlorin | 31.9 | 35.0 | 53.9 | 601.5 | 7.60 |
| Iron chlorin | 31.5 | 34.6 | 53.9 | 586.9 | 4.99 |
| Control | 30.9 | 33.6 | 53.8 | 559.0 | -- |

When sprayed at 3 mg/mu, the iron chlorin and the potassium salt thereof exhibited a significant yield-increasing effect, but the potassium salt had a better yield-increasing effect than iron chlorin.

Example 92: Experiment of controlling diseases in tobacco by spraying sodium iron chlorin

Site: Wujiang Village, Tianwen Town, Weng'an County, Guizhou Province.

Variety: Yunyan 87. The transplanting was conducted on May 13 with a plant spacing of 100 cm and a plant spacing 50 cm.

Samples: sodium iron chlorin, an iron chlorin powder (positive control), a lentinan aqueous solution (positive control), and clear water (blank control).

Method: 6 groups were set as follows: first to third treatments: 3 sodium iron chlorin solutions with different concentrations; a first control: an iron chlorin powder solution; a second control: a 0.5% lentinan aqueous solution; and a third control: the blank control.

For each treatment, 4 plots relatively scattered were selected from the field, with each plot of 50 m$^2$. An agent was sprayed twice around May 28 and June 4 during a root-spreading stage (12 leaves to 14 leaves) after tobacco transplanting, respectively. A same spraying volume (30 L/mu) was adopted for each treatment. During the spraying operation, each leaf surface should be sprayed evenly without dead corners left.

On July 15, tobacco plants in all treatment plots were surveyed. An incidence grading survey was conducted according to the *Grade and investigation method of tobacco diseases and insect pests* GB/T23222-2008, and an incidence rate and a disease index were counted for each treatment plot. Results were as follows:

Control of diseases in tobacco by spraying sodium iron chlorin

| Group | Agent name | Concentration (ppm) | Disease index | Relative control effect (%) |
|---|---|---|---|---|
| First treatment | Sodium iron chlorin | 0.05 | 8.94 | 65.48 |
| Second treatment | Sodium iron chlorin | 0.10 | 8.77 | 66.13 |
| Third treatment | Sodium iron chlorin | 0.20 | 8.15 | 71.00 |
| First control | Iron chlorin | 0.20 | 8.90 | 65.57 |
| Second control | 0.5% lentinan aqueous solution | 10.0 | 9.23 | 64.36 |
| Third control | Clear water | 0 | -- | -- |

Results showed that sodium iron chlorin could significantly improve the immunity of tobacco to play a role in the resistance of tobacco to mosaic virus and reduce an incidence rate, with a better effect than the positive controls of iron chlorin and lentinan.

Example 93: Experiment of spraying sodium iron chlorin on tobacco to improve a yield

Experimental site: Yutang Village, Huanghua Town, Changsha County, Hunan Province.

Variety: Yunyan 87. The planting was conducted with a density of 16,500 plants/ha, a plant spacing of 50 cm, and a row spacing of 110 cm to 120 cm.

Method: Four spraying concentrations were set. For each treatment, four plots were adopted, with each plot of 20 m$^2$. An agent was sprayed twice on May 8, 2019 and May 15, 2019 during a root-spreading stage (12 leaves to 14 leaves) after tobacco transplanting, respectively, with a water amount of 45 L per mu (spraying amount: 1.35 L/plot).

During harvesting, sampling was conducted to count a plant height, a leaf length, a leaf width, and a single-leaf weight. All tobacco leaves of the plots were baked into flue-cured tobacco. The growth and yield of flue-cured tobacco after each sample (sodium iron chlorin) was sprayed at a specified concentration were counted. Results were as follows:

| Sample concentration (ppm) | Plant height (cm) | Leaf length (cm) | Leaf width (cm) | Weight/leaf (g) | Yield (kg/mu) | Yield increase rate (%) |
|---|---|---|---|---|---|---|
| 0.05 | 114.2 | 76.0 | 28.5 | 8.4 | 133.75 | 9.4 |
| 0.10 | 116.7 | 77.2 | 28.9 | 8.7 | 138.67 | 13.5 |
| 0.20 | 114.3 | 75.6 | 28.4 | 8.4 | 133.59 | 9.3 |
| 0.30 | 114.5 | 74.7 | 28 | 8.3 | 132.54 | 8.5 |
| 0 | 109.3 | 71.7 | 26.9 | 7.9 | 122.21 | -- |

There was a specified relationship between an effect of sodium iron chlorin sprayed on tobacco leaves and a concentration of sodium iron chlorin. At the test concentrations, sodium iron chlorin could significantly increase a single-leaf weight for tobacco, and could increase a leaf width and length and a tobacco yield per mu. However, the concentration of 0.1 ppm was the most conducive to the improvement of a tobacco leaf yield.

Example 94: Experiment of spraying sodium iron chlorophyllin on cotton to control verticillium wilt

Experimental site: Dongfanghong Team No. 1, Wutu Prague Town, Bole City, Xinjiang Uygur Autonomous Region. A fore-rotating crop was cotton.
Cotton variety: Xinluzhong 66. The sowing was conducted on April 21, 2020, with four rows for each mulch film and 15,000 plants per mu. An area of 1 mu was adopted for each treatment.
Samples: sodium iron chlorophyllin, VitaCat (positive control), and clear water (blank control).
Method: A total of five treatments were set, including: the sample in three concentrations; the positive control; and the blank control. An agent was sprayed once at each of a seedling stage (May 28) and a bud stage (June 28) of cotton, where a concentration was adjusted each time. Concentration changes of the sodium iron chlorophyllin and controls (first to fifth treatments) were as follows:

| Treatment | Sample | Concentration at a seedling stage (ppm) | Concentration at a bud stage (ppm) |
|---|---|---|---|
| Blank control | Clear water | 0 | 0 |
| First treatment | Sodium iron chlorophyllin | 0.167 | 0.500 |
| Second treatment | Sodium iron chlorophyllin | 0.500 | 0.188 |
| Third treatment | Sodium iron chlorophyllin | 1.000 | 0.833 |
| Positive control | VitaCat | 66.667 | 100.000 |

An incidence rate of verticillium wilt was surveyed 20 d after the application of each sample. A three-point survey method was adopted for each treatment, and 100 plants were selected at each point. A control effect was surveyed. Results were as follows:

| Treatment | First survey (June 28) | | Second survey (August 10) | |
|---|---|---|---|---|
| | Average diseased plant rate (%) | Control effect (%) | Average diseased plant rate (%) | Control effect (%) |
| Blank control | 3.4 | -- | 8.7 | -- |
| First treatment | 2.2 | 35.29 | 5.6 | 35.63 |
| Second treatment | 2.3 | 32.35 | 4.8 | 44.83 |
| Third treatment | 1.8 | 47.06 | 4.1 | 49.42 |
| Positive control | 2.3 | 32.35 | 4.7 | 45.97 |

The harvesting was conducted on November 15, 2020. Three sampling points were selected for each treatment plot during the harvesting, and each sampling point had an area of 0.01 mu. The growth of cotton was first counted. Then, the harvesting was conducted, and a yield increase rate was calculated. Data was as follows:

| Treatment | Effective cotton boll number in 0.01 mu | Average cotton boll weight (g) | Actual seed cotton yield per mu (kg/mu) | Yield increase rate (%) |
|---|---|---|---|---|
| Blank control | 767.67 | 5.1 | 352.8 | -- |
| First treatment | 812.0 | 5.2 | 380.8 | 7.94 |
| Second treatment | 786.1 | 5.3 | 374.9 | 6..26 |
| Third treatment | 812.0 | 5.3 | 386.9 | 9.66 |
| Positive control | 778.67 | 5.3 | 371.4 | 5.27 |

Results showed that sodium iron chlorophyllin could significantly reduce an incidence rate of verticillium wilt in cotton and increase a yield of cotton, and the effect of sodium iron chlorophyllin to control verticillium wilt in cotton increased with the increase of a spraying concentration of sodium iron chlorophyllin at a seedling stage and was better than an effect of the positive control overall.

Example 95: Impact of potassium iron chlorin on the germination of rice under salt stress

In this experiment, three NaCl concentrations of 0 g/L, 2.5 g/L, and 4.5 g/L + 0.2 ppm potassium iron chlorin and a salt stress + blank control (CK: 4.5 g/L NaCl solution) were set. That is, there were the following 4 treatments in total: (1) 4.5 g/L NaCl solution (CK); (2) 0 g/L NaCl solution-0.2 ppm potassium iron chlorin solution, (3) 2.5 g/L NaCl solution-0.2 ppm potassium iron chlorin solution, and (4) 4.5 g/L NaCl solution-0.2 ppm potassium iron chlorin solution.
The selected full seeds of rice (variety: Nanjing 9108) were soaked in a sodium hypochlorite solution to allow disinfection for 2 min to 3 min, and then rinsed with clear water twice to three times. After the rinsing, 50 full seeds were selected and fully immersed in a prepared seed-soaking solution at 25°C for 48 h. The soaked seeds were transferred to a plastic box with two paper towel layers tiled. The germination bed was moistened with a salt solution at a concentration corresponding to each treatment. The seeds were incubated in a 25°C incubator with a light cycle of 12 h light/12 h dark and a humidity of 70%, during which the germination bed was always kept moist. The germination of rice seeds and the growth of seedlings were observed and recorded day by day. A germination rate of each treatment was recorded on day 7, and a number of normal seedlings was counted on day 14. 10 seeds were randomly selected from each treatment to measure a seedling length, a root length, a root number, a root weight, and a seedling weight. The germination energy was counted on day 5 and a germination rate was counted on day 14. Results were shown in the following tables:

Table 5 Impacts of sample solutions on the germination of rice under salt stress at different concentrations

| NaCl concentration (g/L) | Sample concentration (ppm) | Germination energy (%) | Germination rate (%) | Average germinati on time (D) | Germinati on index (%) | Vitalit y index (%) |
|---|---|---|---|---|---|---|
| 0 | 0.2 | 98.0 | 98.0 | 3.10 | 52.21 | 16.29 |
| 2.5 | 0.2 | 96.7 | 97.3 | 3.28 | 49.99 | 14.38 |
| 4.5 | 0.2 | 97.3 | 97.3 | 3.16 | 50.89 | 15.12 |
| 4.5 | 0 | 94.7 | 95.3 | 3.20 | 49.59 | 11.77 |

Table 6 Impacts of sample solutions on the root system of rice under salt stress at different concentrations

| NaCl concentration (g/L) | Sample concentration (ppm) | Root length (cm) | Root number (roots/plant) | Dry root weight (g/plant) |
|---|---|---|---|---|
| 0 | 0.2 | 7.00 | 8.67 | 0.0587 |
| 2.5 | 0.2 | 4.86 | 7.70 | 0.0473 |
| 4.5 | 0.2 | 5.00 | 7.17 | 0.0480 |
| 4.5 | 0 | 4.75 | 6.11 | 0.0383 |

Table 7 Impacts of sample solutions on the growth of rice seedlings under salt stress at different concentrations

| NaCl concentration (g/L) | Sample concentration (ppm) | Dry seedling weight (g) | Seedling height (cm) | First leaf length (cm) |
|---|---|---|---|---|
| 0 | 0.2 | 0.0543 | 6.83 | 3.84 |

(continued)

| NaCl concentration (g/L) | Sample concentration (ppm) | Dry seedling weight (g) | Seedling height (cm) | First leaf length (cm) |
|---|---|---|---|---|
| 2.5 | 0.2 | 0.0547 | 6.68 | 3.79 |
| 4.5 | 0.2 | 0.0580 | 6.71 | 3.88 |
| 4.5 | 0 | 0.0456 | 4.77 | 2.71 |

Table 8 Impacts of sample solutions on a root-shoot ratio and a seedling-promoting rate of rice under salt stress at different concentrations

| NaCl concentration (g/L) | Sample concentration (ppm) | Root-shoot ratio | Increase (%) | Root weight-promot ing speed (mg/d) | Increase (%) | Seedling weight-promot ing rate (mg/d) | Increase (%) |
|---|---|---|---|---|---|---|---|
| 0 | 0.2 | 1.082 | 25.31 | 4.19 | 53.04 | 3.88 | 19.27 |
| 2.5 | 0.2 | 0.865 | 0.15 | 3.38 | 23.48 | 3.90 | 20 |
| 4.5 | 0.2 | 0.836 | -3.17 | 3.43 | 25.22 | 4.14 | 27.32 |
| 4.5 | 0 | 0.864 | 0 | 2.74 | 0 | 3.25 | 0 |

Results showed that potassium iron chlorin exhibited excellent stress resistance, could promote the germination of rice seeds, improve the germination rate and viability, promote the rooting of seedlings and the growth of aboveground parts of plants, increase the root length, and promote the increase of seedling and root weights under salt stress, and could significantly alleviate the harm of salt stress to plants.

Example 96: Experiment of spraying sodium iron chlorophyllin on flooded rice

Site: riverside irrigated cropland in Xiaosijiao Village, Gushu Town, Dangtu County, Ma'anshan City, Anhui Province.

Rice variety: Nanjing 9108.

Waterlogging: On July 10, 2021, an area of 20 mu was flooded. The floodwater receded on July 20. During the 10 d of waterlogging, the plant was totally submerged for nearly 4 d and was mostly submerged for nearly 6 d. After the floodwater receded, rice plants were weak and had yellow and drooping leaves.
Method: A 10.0 ppm sodium iron chlorophyllin solution was sprayed on 10 mu of land through aerial application at an average amount of 1.2 L/mu (sodium iron chlorophyllin amount: 12.0 mg/mu). 10 mu of land was taken as a blank control (0 mg/mu).
Results: On October 20, 2021, the harvesting was conducted. A yield of the control field was 320 kg/mu (moisture content: 16.5%), and a yield of the treatment field was 720 kg/mu (moisture content: 16.5%), indicating that sodium iron chlorophyllin exhibited a very prominent effect when sprayed.

Example 97: Experiment of spraying potassium iron chlorin on drought rice seedlings

Samples: potassium iron chlorin and clear water (control).
Rice variety: Huiliangyou 473.
Method: Full rice seeds were selected, disinfected with 6% sodium hypochlorite for 2 min to 3 min, then rinsed with water, soaked in clear water at room temperature (30±1°C) until the germination was started, and then transferred to seedling trays to raise seedlings. About 15 d later, rice seedlings with consistent growth were selected and transferred into buckets to allow further growth, with 3 holes per bucket and 2 plants per hole. A soil was a clay loam. Before transplanting, the soil was crushed, sieved, and thoroughly mixed. The buckets each had a height of 30 cm and an inner diameter of 30 cm. The water, fertilizers, pests, and diseases during the cultivation in the seedling trays were uniformly managed. A potassium iron chlorin sample was prepared into solutions of 2 concentrations, and then sprayed on transplanted rice seedlings.
Drought model: The transplanting was conducted on August 18, 2020. The next day (August 19), the natural drought and sample-spraying treatment were started for rice seedlings. A soil moisture content was determined by the NY/T 52-1987 method. A specific scheme and drought model results were shown in Table 9:

Table 9 Experimental factors and levels of drought stress for rice

| Treatment | Sample concentration (ppm) | Soil moisture content (%) | | |
| --- | --- | --- | --- | --- |
| | | Day 0 | Day 3 | Day 6 |
| CK | 0 | 26.0 | 10.0 | 6.0 |
| 1 | 0.05 | 26.0 | 10.0 | 8.0 |
| 2 | 0.002 | 26.0 | 6.0 | 7.0 |

As shown in the table, after the natural drought, the soil moisture content changed from the normal (moisture content: 26.0%) on day 0 to mild drought (moisture content: 6.0% to 10.0%) on day 3, and aggravated to moderate drought (moisture content: 6.0% to 8.0%) on day 6.

After the drought stress and sample-spraying were started at a tillering stage (September 19) of rice, a chlorophyll content (mg/g) in leaves was detected twice on day 3 and day 6 (September 21 and September 25), respectively. Results were shown in Table 10 below:

Table 10 Chlorophyll contents and changes thereof in rice leaves under simulated drought conditions

| Sample concentration (ppm) | Day 3 | | Day 6 | |
| --- | --- | --- | --- | --- |
| | Content (mg/g) | Change rate (%) | Content (mg/g) | Change rate (%) |
| 0 | 3.37 | -- | 2.7 | -- |
| 0.002 | 3.69 | 9.49 | 3.86 | 42.96 |
| 0.05 | 4.37 | 29.67 | 4.42 | 63.70 |

Results: A chlorophyll content in rice leaves gradually decreased over time under drought conditions (blank treatment), indicating that the drought had a significant impact on a change of a chlorophyll content in rice leaves. However, a chlorophyll content in rice leaves treated with the potassium iron chlorin solution increased over time, indicating that potassium iron chlorin could maintain a chlorophyll content and delay the decomposition of chlorophyll under drought stress and exhibited obvious stress resistance.

Example 98: *In vitro* experiment of inducing the resistance of a plant to phytophthora blight with sodium iron chlorin

Experimental method: *Nicotiana benthamiana* leaves were injected with sterilized ultrapure water (DDW), 20.0 ppm sodium iron chlorin, and 2.0 ppm sodium iron chlorin, and then inoculated with a fungal cake of *Phytophthora capsici* LT263 on day 7 and day 10 after the injection. At 36 h after the inoculation, results were observed.

Results: Compared with the DDW treatment, the 20.0 ppm and 2.0 ppm sodium iron chlorin treatments of *Nicotiana benthamiana* leaves for 7 d and 10 d could significantly reduce a lesion area produced after the LT263 inoculation.

Conclusion: The 7 d or 10 d treatment of *Nicotiana benthamiana* with the sodium iron chlorin solution could effectively attenuate/reduce the infection of *Phytophthora capsici*.

Example 99: Pot experiment of sodium iron chlorin to induce the resistance of pepper to phytophthora blight Test medium

V8 medium: 1 g of $CaCO_3$ was added per 100 mL of a V8 vegetable juice medium, and then centrifugation was conducted at 6,000 r/min for 10 min to produce a supernatant. The supernatant was collected and 10-fold diluted, then 15% of an agar powder was added, and sterilization was conducted at 121°C for 20 min.

Test seeds: Sujiao No. 5, which was selectively bred by the Vegetable Research Institute of Jiangsu Academy of Agricultural Sciences.

Preparation of *Phytophthora capsici* zoospores: Fungal discs were collected at an edge of a newly-activated *Phytophthora capsici* plate. 6 mm fungal discs were picked with a needle and transferred to an empty Petri dish with a diameter of 9 cm, with fungi facing up and 10 fungal discs for each plate. 15 mL of sterilized water was added to the Petri dish, and the Petri dish was placed in a clean bench under light. The water was changed once every 30 min for 3 times in total. The remaining wastewater was removed by a pipette, and then 10 mL of the V8 liquid medium was added. The Petri dish was incubated in a 25°C incubator for 24 h in the dark to induce the production of a large number of sporangiospores.

Then the Petri dish was placed in a 4°C refrigerator for 30 min and then incubated in a 25°C incubator for 30 min, such that a large number of zoospores were released. 10 μL of a sample was collected, and observed and counted with a hemocytometer under a microscope. The remaining sample was collected for later use.

Determination of a control effect of sodium iron chlorin for phytophthora blight in potted plants

In the experiment, the following 2 treatments were set in total:

control group (CK): clear water was sprayed; and
treatment group: a 2.0 ppm sodium iron chlorin solution was sprayed.

Pepper seedlings at a 4-6 leaf stage were transplanted, with one plant in each pot. 3 d after being treated with the sodium iron chlorin solution, the pepper seedlings were inoculated with *Phytophthora capsici* spores at an inoculum size of about $1 \times 10^5$ spores per seedling. 3 replicates were set for each treatment, and 18 pots were adopted for each replicate. After the treatment, the observation was conducted regularly once a day. An incidence rate of pepper was recorded, and a disease index and a control efficiency were counted.

According to the method in "Specification for Phytophthora Blight Monitoring and Investigation" (NY/T2060.1-2011), the phytophthora blight in plants was investigated. An incidence rate, a disease index, and a control effect were calculated (Ye Minshuo et al., 2019):

$$\text{incidence rate = number of diseased plants/total number of plants} \times 100\%;$$

disease index = [(number of diseased plants at all levels $\times$ relative disease level)/(total number of investigated plants $\times$ 5) $\times$ 100%;

and

$$\text{control effect = [(control disease index - treatment disease index)/control disease index]} \times 100\%.$$

Extraction of total RNA from plants

At 12 h, 24 h, 36 h, 48 h, 60 h, and 72 h of the treatment with sodium iron chlorin, 0.2 g of pepper leaves (a sample) was collected for RNA extraction. The extraction of RNA was conducted by a TRIzol method. A sample was added to a 2 mL centrifuge tube, liquid nitrogen was added, and grinding was fully conducted. 1 mL of a TRIzol reagent (Thermo Fisher, USA) was added, thorough mixing was conducted, and the 2 mL centrifuge tube was allowed to stand for 5 min. 200 μL of chloroform was added, and the 2 mL centrifuge tube was shaken for thorough mixing, then allowed to stand for 5 min, and centrifuged at 12,000 g and 4°C for 15 min to produce a first supernatant. The first supernatant was transferred to a 1.5 mL centrifuge tube, and an equal volume of pre-cooled isopropanol was added. The 1.5 mL centrifuge tube was shaken for thorough mixing, allowed to stand at -80°C for 30 min, and centrifuged at 12,000 g and 4°C for 10 min to produce a second supernatant. The second supernatant was discarded, and 75% alcohol was added. The 1.5 mL centrifuge tube was centrifuged at 12,000 g and 4°C for 5 min to produce a precipitate. The precipitate was washed twice, then dissolved in 30 μL of 0.1% DEPC-treated water, and stored at -80°C.

Synthesis of a first strand of cDNA and quantitative fluorescence polymerase chain reaction (PCR)

The first strand of cDNA was synthesized with an EasyScript®One-Step gDNA Removal and cDNA Synthesis SuperMix kit (TRANS, Beijing) with the residual genomic DNA in an RNA template removed, where a total system was of 20 μL. RNase-free Water was added to 1,000 ng of total RNA to 7 μL, and then 1 μL of Random Primer (N9) was added to produce a mixture of 8 μL in total. After being thoroughly mixed, the mixture was incubated at 65°C for 5 min and placed on ice for 2 min. Then 10 μL of 2×ES Reaction Mix, 1 μL of EasyScript®RT/RI Enzyme Mix, and 1 μL of gDNA Remover were added, and thorough mixing was gently conducted to produce the system. The system was incubated at 25°C for 10 min, then incubated at 42°C for 15 min, heated at 85°C for 5 s to allow inactivation, and then stored at 4°C. cDNA was diluted 40-fold and then store at -20°C for later use.

The expression of disease resistance-related genes PR1, WRKY40, WRKY53, ACCO, and GST in pepper leaves treated with sodium iron chlorin for different times was detected by real-time PCR.

Names and sequences of primers for real-time quantitative PCR (qRT-PCR)

| Primer name | Primer use | Amplification length (bp) | Primer sequence (Abbasi et al., 2020) |
|---|---|---|---|
| PR1-F | | 184 | 5'-CCAGGTAATTGGAGAGGAC-3' |
| PR1-R | | | 5'-CTCCAGTTACTGCACCAT-3 |
| WRKY40-F | | 165 | 5'-CCAGCCTACTTTCCAACT-3' |
| WRKY40-R | qRT-PCR | | 5'-CTCCTCAAGACCGCTATC-3' |
| WRKY53-F | | 175 | 5'-TTGTCTCTCCTACAACCC-3' |
| WRKY53-R | | | 5'-CGTTCAAGTGGAAACTCC-3' |
| ACCO-F | | 160 | 5'-AGGAGCCTAGGTTTGAAG-3' |
| ACCO-R | | | 5'-CTCCACACCATTAGCAAC-3' |
| GST-F | | 168 | 5'-GACTTTGGCTGGGAGTTT-3' |
| GST-R | | | 5'-CTTGGAAACGAGCTTGGA-3' |
| ACT-F | Reference gene | 228 | 5'-TGTTATGGTAGGGATGGGTC-3' |
| ACT-R | | | 5'-TTCTCTCTATTTGCCTTGGG-3' |

With cDNA as a template, amplification was conducted on a ROCHE LightCycler® 96 real-time qRT-PCR instrument. An amplification system was as follows: 10 μL of 2×PerfectStart TM Green Green qPCR SuperMix (TRANS, Beijing), 0.4 μL (10 μmol/L) of each of upstream and downstream primers, 1 μL of cDNA, and 8.2 μL of Nuclease-free Water for making up to a total volume of 20 μL. A reaction procedure was as follows: pre-denaturation at 95°C for 180 s; 95°C for 10 s and 60°C for 30 s, with 40 cycles; 95°C for 10 s, 65°C for 10 s, and 97°C for 1 s; and finally cooling at 37°C for 30 s. The specificity of a reaction system was determined by a dissolution curve and a fluorescence value change curve. The experiment was repeated 3 times. A relative expression level of a gene was calculated by 2-ΔΔCt. Results were as follows:

| Treatment | Disease index | Control effect (%) |
|---|---|---|
| Sodium iron chlorin | 24.36 | 71.89 |
| Clear water control | 86.67 | |

After the treatment of sodium iron chlorin, an incidence rate of phytophthora blight was significantly reduced, and a control effect reached 71.89%, indicating that sodium iron chlorin had a strong plant immunity-inducing activity at a concentration of 2.0 ppm.

Example 100: Alleviation of herbicide damage to significantly increase a rice yield

Site: Mahu Village, Xuyi County, Huai'an City, Jiangsu Province.
Rice variety: Jinjing 818.
Sample: sodium iron chlorin (0.25 ppm aqueous solution).
Time: June to November in 2021
Plot conditions: A fore-rotating crop was wheat. There was a consistent soil fertility and a same field management.
Method: A treatment plot of 1.5 mu and a control plot of 1.5 mu were set. The sample was sprayed twice after the herbicide damage occurred in rice sprayed with a herbicide. Clear water was adopted as a blank control. Specific operations were as follows:
On June 20 when weeds had 3 leaves and rice had 3 to 3.5 leaves, the treatment plot and the control plot both were treated with 4% imazamox at 200 mL/mu and 10% pyribenzoxim at 40 mL/mu by a stem and leaf spray method, with 30 kg of water per mu. One week later, a weeding effect was prominent, but rice underwent obvious pesticide damage. 10 d after the herbicide application (June 30), the treatment plot was sprayed with the sample at 7.5 mg/mu by a stem and leaf spray method, with a solution of 30 L per mu. The control plot was sprayed with clear water. At an inflorescence emergence stage of rice (August 25), the spraying was conducted once again by the same method.
Results: The harvesting was conducted on November 10. Yield data was as follows:

| Treatment | Ear number/mu | Grain number/ear | Seed set rate | Filled grain number/ear | Thousand-grain weight (g) | Theoretical yield (kg/mu) | Actual yield (kg/mu) |
|---|---|---|---|---|---|---|---|
| Clear water | 256238.6 | 111.5 | 0.8839 | 98.55 | 29.95 | 756.5 | 550 |
| Sample | 275945.5 | 104.5 | 0.9344 | 97.64 | 30.30 | 816.8 | 675 |

It can be seen that a weeding effect was prominent in the rice plots, and there was no obvious difference between the treatment plot and the control plot, but a specified damage effect affecting the growth of a crop was produced. Rice in the plot sprayed with the sodium iron chlorin solution had a better growth status than rice in the blank control. In the plot sprayed with the sodium iron chlorin solution, an ear number per mu, a filled grain number per ear, and a thousand-grain weight all increased significantly, a yield increased significantly, and a yield per mu increased by 22% or more. It can be seen that the spraying of the sodium iron chlorin solution not only had no impact on a weeding effect, but also could significantly reduce the adverse effects of a herbicide to increase a yield per mu.

**Claims**

1. A porphin salt, comprising a salt of a porphin compound or a salt of a chlorin compound.

2. The porphin salt according to claim 1, **characterized in that** the salt of the porphin compound comprises a salt of protoporphyrin or a salt of a protoporphyrin chelate; and the salt of the chlorin compound comprises a salt of pheophorbide or a salt of a pheophorbide chelate.

3. The porphin salt according to claim 1, **characterized in that** the porphin compound is selected from a group consisting of protoporphyrin, chlorhematin, and hydroxyhematin; and the chlorin compound is selected from a group consisting of pheophorbide, iron chlorin (iron chlorophyllin chloride), iron hydroxychlorophyllin, iron chlorophyllin, zinc chlorophyllin, and copper chlorophyllin.

4. The porphin salt according to claim 1, **characterized in that** the salt of the porphin compound is prepared through a salt-producing reaction of acidic protoporphyrin or a chelate thereof with a metal ion; and the salt of the chlorin compound is prepared through a salt-producing reaction of acidic pheophorbide or a chelate thereof with a metal ion.

5. The porphin salt according to claim 1, **characterized in that** the salt of the porphin compound comprises a monovalent, divalent, or trivalent metal ion salt of the porphin compound; and the salt of the chlorin compound comprises a monovalent, divalent, or trivalent metal ion salt of the chlorin compound.

6. The porphin salt according to claim 1, **characterized in that** the salt of the porphin compound comprises a sodium, potassium, ammonium, magnesium, calcium, ferrous iron, ferric iron, zinc, manganese, or copper salt of the porphin compound; and the salt of the chlorin compound comprises a sodium, potassium, ammonium, magnesium, calcium, ferrous iron, ferric iron, zinc, manganese, or copper salt of the chlorin compound.

7. The porphin salt according to claim 1, **characterized in that** the porphin salt comprises:

   a potassium, ammonium, magnesium, calcium, ferrous iron, ferric iron, zinc, manganese, and copper salt of protoporphyrin,
   a sodium, potassium, ammonium, magnesium, calcium, ferrous iron, ferric iron, zinc, manganese, and copper salt of chlorhematin,
   a potassium, ammonium, magnesium, calcium, ferrous iron, ferric iron, zinc, manganese, and copper salt of hydroxyhematin,
   a sodium, potassium, ammonium, magnesium, calcium, ferrous iron, ferric iron, zinc, manganese, and copper salt of iron chlorin (iron chlorophyllin chloride),
   a sodium, potassium, ammonium, magnesium, calcium, ferrous iron, ferric iron, zinc, manganese, and copper salt of iron hydroxychlorophyllin,
   an ammonium, ferrous iron, ferric iron, and copper salt of iron chlorophyllin,
   a potassium, ammonium, magnesium, ferrous iron, ferric iron, zinc, manganese, and copper salt of chlorin (pheophorbide),

a potassium, ammonium, magnesium, calcium, ferrous iron, ferric iron, zinc, manganese, and copper salt of zinc chlorophyllin, and
an ammonium, magnesium, calcium, ferrous iron, ferric iron, zinc, manganese, and copper salt of copper chlorophyllin.

8. A preparation method of the porphin salt according to any one of claims 1 to 7, **characterized by** comprising: allowing an acidic porphin compound or an acidic chlorin compound to react with a monovalent metal hydroxide to produce a precipitate, and separating the precipitate to obtain a monovalent metal ion salt of the porphin compound or the chlorin compound; or allowing a monovalent metal salt of the porphin compound or the chlorin compound to react with a soluble polyvalent metal ion salt to produce a precipitate, and separating the precipitate to obtain a polyvalent metal ion salt of the porphin compound or the chlorin compound.

9. The preparation method of the porphin salt according to claim 8, **characterized in that** the monovalent metal hydroxide is selected from a group consisting of sodium hydroxide, potassium hydroxide, and ammonia water; and in a preparation process of the polyvalent metal ion salt of the porphin compound or the chlorin compound, the monovalent metal salt of the porphin compound or the chlorin compound is selected from a group consisting of sodium and potassium salts of the porphin compound or the chlorin compound; and the soluble polyvalent metal ion salt comprises a sulfate, a hydrochloride, or a nitrate of a divalent or trivalent metal ion.

10. The preparation method of the porphin salt according to claim 8, **characterized in that**
a preparation process of the monovalent metal ion salt of the porphin compound or the chlorin compound comprises following steps:

preparing the acidic porphin compound or the acidic chlorin compound into a solution with an organic solvent, and mixing with an organic solvent of a monovalent metal ion salt for precipitation; or subjecting the acidic porphin compound or the acidic chlorin compound and the monovalent metal ion salt to a salt-producing reaction in an aqueous solution, and conducting precipitation with an organic solvent; or preparing the acidic porphin compound or the acidic chlorin compound into a solution with an organic solvent, and introducing a dry ammonia gas into the solution to produce a precipitate; and finally conducting filtration, washing, and drying; and
a preparation process of the polyvalent metal ion salt of the porphin compound or the chlorin compound comprises following steps:
mixing an aqueous solution of the monovalent metal salt of the porphin compound or the chlorin compound with an aqueous solution of the soluble polyvalent metal ion salt, and conducting precipitation, filtration, washing, and drying.

11. A use of the porphin salt according to any one of claims 1 to 6 as a plant growth regulator or a plant immunity inducer.

12. The use according to claim 11, **characterized in that** the porphin salt comprises:

a sodium, potassium, ammonium, magnesium, calcium, ferrous iron, ferric iron, zinc, manganese, and copper salt of protoporphyrin,
a sodium, potassium, ammonium, magnesium, calcium, ferrous iron, ferric iron, zinc, manganese, and copper salt of chlorhematin,
a sodium, potassium, ammonium, magnesium, calcium, ferrous iron, ferric iron, zinc, manganese, and copper salt of hydroxyhematin,
a sodium, potassium, ammonium, magnesium, calcium, ferrous iron, ferric iron, zinc, manganese, and copper salt of iron chlorin,
a sodium, potassium, ammonium, magnesium, calcium, ferrous iron, ferric iron, zinc, manganese, and copper salt of iron hydroxychlorophyllin,
a sodium, potassium, ammonium, magnesium, calcium, ferrous iron, ferric iron, zinc, manganese, and copper salt of iron chlorophyllin,
a sodium, potassium, ammonium, magnesium, calcium, ferrous iron, ferric iron, zinc, manganese, and copper salt of chlorin,
a sodium, potassium, ammonium, magnesium, calcium, ferrous iron, ferric iron, zinc, manganese, and copper salt of zinc chlorophyllin, and
a sodium, potassium, ammonium, magnesium, calcium, ferrous iron, ferric iron, zinc, manganese, and copper salt of copper chlorophyllin.

13. The use according to claim 11, **characterized by** comprising: applying the porphin salt through spraying, smearing, seed soaking, aerial application, ear soaking, irrigation, or sprinkling to a plant requiring growth regulation or stress resistance enhancement or an environment in which the plant grows to regulate growth of the plant or enhance stress resistance of the plant.

14. The use according to claim 11, **characterized in that** the porphin salt is applied at a concentration/content of 0.001 ppm to 10 ppm.

15. The use according to claim 11, **characterized in that** in case of seed soaking and field irrigation, the porphin salt is applied at a concentration/content of 0.001 ppm to 0.1 ppm; and in case of foliar spraying, the porphin salt is applied at a concentration/content of 0.01 ppm to 10 ppm.

16. The use according to claim 11, **characterized in that** effects of the plant growth regulator comprise promoting seed germination, improving a germination rate, increasing a root length, promoting growth of a root, enhancing immunity and stress resistance of a plant, accelerating growth of a seedling, increasing a chlorophyll content, delaying premature senescence of a plant, and improving a yield and a quality.

17. The use according to claim 11, **characterized in that** effects of the plant immunity inducer comprise improving stress resistance of a plant and increasing a yield of a crop.

18. A use of a composition comprising the porphin salt according to any one of claims 1 to 6 as a plant growth regulator or a plant immunity inducer.

19. The use according to claim 18, **characterized in that** the porphin salt comprises:

a sodium, potassium, ammonium, magnesium, calcium, ferrous iron, ferric iron, zinc, manganese, and copper salt of protoporphyrin,
a sodium, potassium, ammonium, magnesium, calcium, ferrous iron, ferric iron, zinc, manganese, and copper salt of chlorhematin,
a sodium, potassium, ammonium, magnesium, calcium, ferrous iron, ferric iron, zinc, manganese, and copper salt of hydroxyhematin,
a sodium, potassium, ammonium, magnesium, calcium, ferrous iron, ferric iron, zinc, manganese, and copper salt of iron chlorin,
a sodium, potassium, ammonium, magnesium, calcium, ferrous iron, ferric iron, zinc, manganese, and copper salt of iron hydroxychlorophyllin,
a sodium, potassium, ammonium, magnesium, calcium, ferrous iron, ferric iron, zinc, manganese, and copper salt of iron chlorophyllin,
a sodium, potassium, ammonium, magnesium, calcium, ferrous iron, ferric iron, zinc, manganese, and copper salt of chlorin,
a sodium, potassium, ammonium, magnesium, calcium, ferrous iron, ferric iron, zinc, manganese, and copper salt of zinc chlorophyllin, and
a sodium, potassium, ammonium, magnesium, calcium, ferrous iron, ferric iron, zinc, manganese, and copper salt of copper chlorophyllin.

FIG. 1

FIG. 2

## INTERNATIONAL SEARCH REPORT

| International application No. |
| --- |
| **PCT/CN2022/117298** |

| A. | CLASSIFICATION OF SUBJECT MATTER |
| --- | --- |

C07D487/22(2006.01)i; C07D491/22(2006.01)i; A01N43/90(2006.01)i; A01P21/00(2006.01)i

According to International Patent Classification (IPC) or to both national classification and IPC

| B. | FIELDS SEARCHED |
| --- | --- |

Minimum documentation searched (classification system followed by classification symbols)

C07D,A01N,A01P

Documentation searched other than minimum documentation to the extent that such documents are included in the fields searched

Electronic data base consulted during the international search (name of data base and, where practicable, search terms used)

CNKI, VCN, DWPI, VEN, ENTXT, WOTXT, REGISTRY, CAPLUS: 根据权利要求1进行的子结构检索, search for substructure according to claim 1, 卟吩, 二氢卟吩, 盐, 原卟啉, 氯化血红素, 羟基血红素, 二氢卟吩铁, 氯化叶绿酸铁, 羟基叶绿酸铁, 脱镁叶绿酸, 叶绿酸锌, 叶绿酸铜, 钠, 钾, 铵, 镁, 钙, 铁, 亚铁, 锌, 锰, 铜, Porphines, chlorins, salts, protoporphyrins, hemin, chlorin ferric, ferric hydroxychlorophyllin, pheophorbide, zinc chlorophyllin, copper chlorophyllin, sodium, potassium, ammonium, magnesium, calcium, ferric, ferrous, zinc, manganese, copper, Na, K, Mg, Ca, Fe, Zn, Mn, Cu

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2021163782 A1 (SUNCOR ENERGY INC.) 26 August 2021 (2021-08-26) description, embodiments 25-26, tables 25-26, and paragraphs 108-112 and 0124-0195 | 1-19 |
| X | JP H04202193 A (SATO YAKUGAKU KENKIYUUSHIYO KK) 22 July 1992 (1992-07-22) description, embodiment 2 | 1-10 |
| X | CN 110585215 A (SUN YAT-SEN UNIVERSITY) 20 December 2019 (2019-12-20) description, embodiments 1 to 3 | 1-7 |
| A | Paneque, Armando et al. "Solid State Reactions of Hemin with Basic Substances: Formation of bis and Mixed Complexes" *Structural Chemistry*, Vol. 14, No. (6), 31 December 2003 (2003-12-31), pp. 551-558 entire document | 1-19 |
| A | CN 102273467 A (NANJING NORMAL UNIVERSITY et al.) 14 December 2011 (2011-12-14) description, embodiment 5 | 1-19 |

☑ Further documents are listed in the continuation of Box C.     ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **18 May 2023** | **05 June 2023** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2022/117298** |

**C.    DOCUMENTS CONSIDERED TO BE RELEVANT**

| Category* | Citation of document, with indication, where appropriate, of the relevant passages | Relevant to claim No. |
|---|---|---|
| X | CN 102285992 A (NANJING NORMAL UNIVERSITY; NANJING BAITE BIOLOGICAL ENGINEERING CO., LTD.) 21 December 2011 (2011-12-21)<br>description, paragraph 0024, and embodiments 15-26 | 1-19 |
| X | Hunt, Jerry E. et al. "Application of Californium-252 Plasma Desorption Mass Spectroscopy to Chlorophyll Studies"<br>*International Journal of Mass Spectrometry and Ion Physics,*<br>Vol. vol. 53, 31 December 1983 (1983-12-31), 45-58<br>page 52, Table 1 | 1-7 |
| X | 彭亦如 (PENG, Yiru). "肿瘤光动力治疗药物研究进展 (一) (Non-official translation: Tumor Photodynamic Therapy Drug Research Progress (I))"<br>福建医药杂志 (Fujian Medical Journal), Vol. vol. 25, 20 June 2003 (2003-06-20), 124-125<br>page 125, right-hand column, paragraph 2 | 1-7 |
| X | 刘淑萍等 (LIU, Shuping et al.). "金属叶绿素衍生物的制备及光谱研究进展 (The Preparation and Spectrum Progress in the Study of Metal Chlorophyll Derivatives)"<br>唐山师范学院学报 (Journal of Tangshan Teachers College),<br>Vol. 36, No. (2), 20 March 2014 (2014-03-20), 28-32<br>page 30, right-hand column, last paragraph | 1-7 |
| X | CN 114287438 A (JI, Hongjin) 08 April 2022 (2022-04-08)<br>description, table 1 | 1-7, 11-13, 16-19 |
| X | 孙彩云等 (SUN, Caiyun et al.). "金属叶绿素衍生物的研究进展 (Progress on the Research of Metal-Substituted Chlorophyll Derivatives)"<br>唐山师范学院学报 (Journal of Tangshan Teachers College),<br>Vol. 30, No. (2), 20 March 2008 (2008-03-20), 唐山师范学院学报 (Journal of Tangshan Teachers College)<br>page 56, right-hand column, paragraph 1 | 1-7 |
| X | CN 110090214 A (WUHAN UNITED PHARMACEUTICAL CO., LTD.) 06 August 2019 (2019-08-06)<br>description, paragraph 0008 | 1-7 |
| X | CN 1082049 A (BI, Sibin et al.) 16 February 1994 (1994-02-16)<br>description, embodiment 4 | 1-7 |
| X | CN 112203511 A (SUNCOR ENERGY INC.) 08 January 2021 (2021-01-08)<br>description, embodiment 4, table 4B, and paragraphs 0015-0017 | 1-7, 11-13, 16-19 |
| A | CN 101045730 A (NORTH CHINA UNIVERSITY OF SCIENCE AND TECHNOLOGY) 03 October 2007 (2007-10-03)<br>entire document | 1-19 |
| X | CN 113631039 A (SUNCOR ENERGY INC.) 09 November 2021 (2021-11-09)<br>claims 1-141 | 1-19 |
| A | CN 112940528 A (HENAN UNIVERSITY OF URBAN CONSTRUCTION et al.) 11 June 2021 (2021-06-11)<br>entire document | 1-19 |
| A | CN 1418880 A (SHANGHAI JINCHI BIOLOGICAL PRODUCTS CO., LTD. et al.) 21 May 2003 (2003-05-21)<br>entire document | 1-19 |
| A | JP S60156690 A (SATO YAKUGAKU KENKYUSHO KK) 16 August 1985 (1985-08-16)<br>entire document | 1-19 |
| A | US 2022248677 A1 (NANJING RUIJIANG BIOLOGICAL ENG CO., LTD.; SHANGHAI INST PHARMACEUTICAL IND;) 11 August 2022 (2022-08-11)<br>entire document | 1-19 |

## INTERNATIONAL SEARCH REPORT

International application No.

**PCT/CN2022/117298**

**Box No. III    Observations where unity of invention is lacking (Continuation of item 3 of first sheet)**

This International Searching Authority found multiple inventions in this international application, as follows:

This International Authority is of the opinion that claims 1-19 comprise a plurality of inventions as follows, the same or corresponding technical feature of which is: a salt of a compound of a porphin or chlorin macrocyclic structure.

Invention 1: a salt of protoporphyrin and a preparation method and use thereof.

Invention 2: a salt of heme chloride and a preparation method and use thereof.

Invention 3: a salt of hydroxyheme and a preparation method and use thereof.

Invention 4: a salt of pheophorbide and a preparation method and use thereof.

Invention 5: a salt of chlorin ferric and a preparation method and use thereof.

Invention 6: a salt of ferric hydroxychlorophyllin and a preparation method and use thereof.

Invention 7: a salt of ferric chlorophyllin and a preparation method and use thereof.

Invention 8: a salt of zinc chlorophyllin and a preparation method and use thereof.

Invention 9: a salt of copper chlorophyllin and a preparation method and use thereof.

However, the same or corresponding technical feature "a salt of a compound of a porphin or chlorin macrocyclic structure" is known in the art, see the prior art: CN 1418880 A (publication date: 21 May 2003) discloses a protoporphyrin disodium salt, CN 109053744 A (publication date: 21 December 2018) discloses compounds of a porphin diether disodium salt, a porphin diether dipotassium salt, a porphin diether dilithium salt and a porphin diether diammonium salt, and CN 101045730 A (publication date: 03 October 2007) discloses an chlorophyll iron-zinc salt. Therefore, the plurality of inventions do not have a same or corresponding special technical feature that defines a contribution which the inventions make over the prior art, do not have a technical relationship therebetween, do not fall within a single general inventive concept, and therefore do not comply with the requirement of unity of invention and do not comply with PCT Rule 13.1.

1. [✓] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**    [ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[✓] No protest accompanied the payment of additional search fees.

Form PCT/ISA/210 (continuation of first sheet) (July 2022)

## INTERNATIONAL SEARCH REPORT
### Information on patent family members

International application No.

**PCT/CN2022/117298**

| Patent document cited in search report | | | Publication date (day/month/year) | Patent family member(s) | | | Publication date (day/month/year) |
|---|---|---|---|---|---|---|---|
| WO | 2021163782 | A1 | 26 August 2021 | CA | 3162683 | A1 | 26 August 2021 |
| | | | | BR | 112022016429 | A2 | 04 October 2022 |
| | | | | US | 2023128730 | A1 | 27 April 2023 |
| | | | | AU | 2020430398 | A1 | 21 July 2022 |
| | | | | AR | 121385 | A1 | 01 June 2022 |
| | | | | EP | 4106524 | A1 | 28 December 2022 |
| JP | H04202193 | A | 22 July 1992 | JP | 2997035 | B2 | 11 January 2000 |
| CN | 110585215 | A | 20 December 2019 | None | | | |
| CN | 102273467 | A | 14 December 2011 | None | | | |
| CN | 102285992 | A | 21 December 2011 | None | | | |
| CN | 114287438 | A | 08 April 2022 | None | | | |
| CN | 110090214 | A | 06 August 2019 | None | | | |
| CN | 1082049 | A | 16 February 1994 | None | | | |
| CN | 112203511 | A | 08 January 2021 | CL | 2020002775 | A1 | 18 December 2020 |
| | | | | US | 2021352889 | A1 | 18 November 2021 |
| | | | | WO | 2019210403 | A1 | 07 November 2019 |
| | | | | CA | 3097038 | A1 | 07 November 2019 |
| | | | | BR | 112020021912 | A2 | 02 March 2021 |
| | | | | MX | 2020011488 | A | 18 February 2021 |
| | | | | EP | 3787401 | A1 | 10 March 2021 |
| | | | | EP | 3787401 | A4 | 26 January 2022 |
| | | | | AU | 2019264145 | A1 | 12 November 2020 |
| | | | | JP | 2021521859 | A | 30 August 2021 |
| CN | 101045730 | A | 03 October 2007 | None | | | |
| CN | 113631039 | A | 09 November 2021 | EP | 3923730 | A1 | 22 December 2021 |
| | | | | EP | 3923730 | A4 | 30 November 2022 |
| | | | | WO | 2020163965 | A1 | 20 August 2020 |
| | | | | CA | 3128734 | A1 | 20 August 2020 |
| | | | | US | 2022132856 | A1 | 05 May 2022 |
| | | | | CL | 2021002022 | A1 | 07 January 2022 |
| | | | | JP | 2022520109 | A | 28 March 2022 |
| | | | | AU | 2020220245 | A1 | 19 August 2021 |
| CN | 112940528 | A | 11 June 2021 | None | | | |
| CN | 1418880 | A | 21 May 2003 | None | | | |
| JP | S60156690 | A | 16 August 1985 | JPH | 0421674 | B2 | 13 April 1992 |
| US | 2022248677 | A1 | 11 August 2022 | WO | 2020258190 | A1 | 30 December 2020 |
| | | | | CA | 3146587 | A1 | 30 December 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102285992 B
- CN 10048884 C
- CN 101045730
- CN 102351867
- CN 102775416
- CN 102796108

### Non-patent literature cited in the description

- Study on Reaction Rates, Equilibrium Constants, and Structures of Metallic Salts of Iron Chlorophyllin. North China University of Science and Technology, March 2017
- Properties and detection methods of chlorhematin. *Chinese Journal of Biochemical Pharmaceutics*, 1993, vol. 66 (4), 58-59
- Preliminary Study on Basic Properties of Iron Chlorin Solution. Journal of Nanjing Normal University. Natural Science Edition, 2020, vol. 43, 143-148
- *Spectroscopy Laboratory*, 2011, vol. 28 (3), 1165-1169
- Preparation of Sodium Chlorhematin. *Chemistry World*, 2004, vol. 10, 540-541
- *Acta Botanica Boreali-Occidentalia Sinica*, 2003, vol. 23 (5), 750-754